(19) Europäisches Patentamt
European Patent Office
Office européen des brevets

(11) Publication number: **0 235 391 B1**

(12) # EUROPEAN PATENT SPECIFICATION

(45) Date of publication of patent specification: **10.06.92**

(51) Int. Cl.[5]: **C07K 13/00**, A61K 39/15, A61K 39/385

(21) Application number: **86117981.0**

(22) Date of filing: **23.12.86**

(54) **Peptides corresponding to antigenic and immunogenic determinants of major neutralizing proteins of rotaviruses.**

(30) Priority: **26.12.85 US 813661**
**03.09.86 US 903325**

(43) Date of publication of application:
**09.09.87 Bulletin 87/37**

(45) Publication of the grant of the patent:
**10.06.92 Bulletin 92/24**

(84) Designated Contracting States:
**BE CH DE FR GB LI NL SE**

(56) References cited:
**WO-A-85/05122**

**Journal 0f Virology, vol. 54, no 3, June 1985, pages 791-797. P R Gunn et al. : "Rotavirus neutralising protein VP7 : Anti-genic determinants investigated by sequence analysis and peptide synthesis"**

**Journal of Virology, vol. 51, no 1, July 1984, pages 97-101. G W Both et al.: "Comparative sequence analysis of rotavirus genomic segment 6 - the gene specifying viral subgroups 1 and 2"**

(73) Proprietor: **The University of Saskatchewan**
**124 Veterinary Road**
**Saskatoon, Saskatchewan S7N 0W0(CA)**

(72) Inventor: **Sabara, Marta I.**
**316-114 Clarence Avenue South**
**Saskatoon,Saskatchewan S7N 1H1(CA)**
Inventor: **Frenchick, Patrick J.**
**722 9th Avenue North**
**Saskatoon,Saskatchewan S7K 2Y9(CA)**
Inventor: **Potter, Andrew A.**
**521 Dalhousie Crescent**
**Saskatoon,Saskatchewan S7H 3S5(CA)**
Inventor: **Ijaz, Mohammad K.**
**11-2901 7th Street East**
**Saskatoon,Saskatchewan S7H 1B1(CA)**
Inventor: **Gilchrist, James E.**
**410 Alberta Avenue**
**Saskatoon,Saskatchewan S7N 1G3(CA)**

(74) Representative: **Goldin, Douglas Michael et al**
**J.A. KEMP & CO. 14, South Square Gray's Inn**
**London WC1R 5EU(GB)**

**EP 0 235 391 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

| | |
|---|---|
| Virology, vol. 144, no. 1, 1985, pages 11-19. S Lopez et al. : "Primary structure of the cleavage site associated with trypsin enhancement of rotavirus SA11 infectivity | |

## Description

The present invention relates to peptide fragments of the major outer capsid neutralizing glycoprotein (virus protein 7 or VP7), an inner or nucleocapsid protein (virus protein 6 or VP6), and an outer shell protein (virus protein 3 or VP3) of rotaviruses and their use as vaccines in birds and mammals including man. The invention further relates to the production of relevant peptide fragments of rotavirus, to the attachment of peptides to carriers and to their use as vaccines. An added feature of the VP3 peptide is its ability to compete with rotavirus for trypsin, thus interfering with infectivity of the virus. Therefore, the VP3 peptide may also have therapeutic use.

### BRIEF DESCRIPTION OF THE DRAWINGS

FIGURE 1 shows the amino acid sequence of rotavirus VP7 glycoprotein the 14K polypeptide is shown in the box.

FIGURE 2 shows the nucleotide sequence of cloned copy of SA-11 gene 6. The (+) sense strand (corresponding to the mRNA) is shown. The predicted amino acid sequence of the protein product is shown and the termination sites are underlined.

FIGURE 3 shows the amino acid sequence of rotavirus VP3 protein. The C486 (bovine) VP3 amino acid sequence was generated in the laboratory. The SA-11 (simian) VP3 amino acid sequence was from prior art.

FIGURE 4 shows the isolation of a bovine rotavirus (BRV) 14k peptide fragment. Lanes A and B illustrate the digestion pattern of the BRV glycoprotein produced with 65 ug of papain/cm of gel and 130 ug of papain/cm of gel, respectively. Lane C illustrates the purified 14K peptide fragment. Lanes represent immunoblot-ELISA reactions with monoclonal antibodies from hybridoma 11D12-6. Lane D illustrates the purified 14k peptide fragment in a silver stained polyacrylamide gel.

FIGURE 5 shows the antibody titers to various preparations of the 14k polypeptide at three different times during the immunization schedule. The upper panel shows total antibody titers as determined by ELISA using double-shelled rotavirus as the antigen. The lower panel shows neutralizing antibody titers as determined by plaque reduction assays. Group A, 14k unconjugated poly-peptide; Group B, 14k BSA-conjugated polypeptide; Group C, purified VP7; Group D, infectious bovine rotavirus (BRV);
Group $E_7$, animals given infectious BRV at 61 days;
Group $E_2$, animals given adjuvant. The immunization protocol is described in more detail in Table 2.

FIGURE 6 shows the immuno-blot ELISA reaction of mouse sera with the C486 bovine rotavirus (BRV) protein profile. Lane P, prebleed; Lane A, group given 14k unconjugated peptide; Lane B, 14k conjugated peptide; Lane C, group given BRV VP7 (38.2k glycoprotein); Lane D, group given infectious virus; Lane E and E2, groups given saline and Freund's complete adjuvant respectively. The immunization schedule for each group is described in more detail in Table 2.

FIGURE 7 shows the cleavage patterns of VP7 by Staph aureus V8 protease, papain, chymotrypsin and cyanogen bromide to locate 14k.

FIGURE 8 shows the ability of synthetic peptides of VP7 to block virus attachment to cells.

FIGURE 9 shows the ELISA antibody titers to synthetic peptides.

FIGURE 10 shows the virus neutralizing antibody titres in mice induced by synthetic peptides to VP7.

FIGURE 11 shows the Immunoprecipitation of [$^{35}$S] methionine-labelled, bovine rotavirus-infected cell lysate by monoclone 1D7 (lane B); 1B4 (lane C); 1B9 (lane D) and 1D10 (lane E). Lane A; is the reaction with rabbit hyperimmune anti-bovine rotavirus. The position of the molecular weight markers are demonstrated on the left hand side.

FIGURE 12 shows the reaction of monoclonal antibodies and monospecific polyclonal antisera with bovine rotavirus polypeptides transferred to nitrocellulose. Lane A, monoclonal antibody 1D7; lane B, 1B4; lane C, 1B9; lane D, 1D10 and Lane E, anti-nucleocapsid monospecific antisera. The molecular weights of the reaction proteins are given on the left hand side.

FIGURE 13 shows the immune-blot ELISA reaction of bovine, simian, pig and human rotavirus VP6 nucleo-capsid proteins with monoclonal antibody 1B4. Lane A, bovine isolate C486; Lane B, procine isolate OSU; Lane C, bovine isolate NCDV; Lane D, human isolate Wa; Lane E, bovine isolate UK; Lane F, human isolate ST4; Lane G, simian isolate SA-11. Human isolates Wa and ST4 belong to subgroup 2, while all other isolates belong to sub-group 1. Molecular weights are indicated on the right hand side and ascites control is designated.

FIGURE 14 shows the reaction of bovine rotavirus VP6 nucleocapsid protein digests with monoclonal antibodies 1D7, 1B4, 1B9 and 1D10 and with monospecific antiserum. The enzyme and quantity used per digest are indicated on the figure.

FIGURE 15 shows partial carboxypeptidase digest and Cyanogen bromide chemical cleavage of the VP6 nucleocapsid protein and immunoblot ELISA reaction with monoclonal antibody 1B9. Lanes A-I represent the autoradiogram of the digests; Lanes A'-I' represent the corresponding immunoblot ELISA reaction using monoclonal antibody 1B9. Lanes A, A' 100 ug carboxypeptidase; B, B', 50 ug carboxypeptidase C, C', 25 ug carboxy-peptidase; D, D', 2.5 ug carboxypeptidase; E, E', .25 ug carboxypeptidase; F, F', undigested nucleocapsid; G, G', 2 h digestion with 50 ug cyanogen bromide; H, H', 1 h digestion with 50 ug cyanogen bromide; I, I', 30 min digestion with 50 ug cyanogen bromide. The positions of molecular weight standards are indicated on the left hand side of the figure.

FIGURE 16 shows the reaction of antiserum to the synthetic peptide 232-256 of VP3 with the rotavirus protein profile. The upper panel (Lane A-H) represents the autoradiograph and the lower panel (Lanes A'-H') represents the immunoblot-ELISA reaction of the protein profile shown in the upper panel with anti-synthetic peptide serum. Lanes A-D represent protein profiles generated in the presence of Laemmli sample buffer containing BME and boiled, and Lanes E-H represent protein profiles generated in the presence of Laemmli sample buffer without BME but boiled. Lanes A, A' and E and E' represent a mixture of 100 ug of synthetic peptide, 2.0 ug double-shelled rotavirus, and .96 ug trypsin. Lanes B and B' and F and F' represent a mixture of 25 ug of synthetic peptide, 2.0 ug of double-shelled rotavirus and .96 ug trypsin. Lanes C and C'; D and D' and G and G' represent 2.0 ug of double-shelled rotavirus and .96 ug trypsin. Lane H and H' represent double-shelled virus. The numbers indicate position of various proteins referred to in the text. Molecular weight markers are indicated on the right hand side.

FIGURE 17 shows the reactivity of synthetic peptide with the VP3 nucleocapsid protein of bovine rotavirus. Lane A represents the protein profile after reacting 2.0 ug of double-shelled virus with 25 ug of synthetic peptide, Lane B represents the same profile as in A after treatment with .96 ug of trypsin. Lane C represents the protein profile after reacting 2.0 ug of double-shelled virus with 100 ug of synthetic peptide and Lane D represents the same profile as in C after treatment with 0.96 ug of trypsin. Lane E represents the protein profile of 2.0 ug of virus treated with 0.96 ug of trypsin and Lane F represents the protein profile of 2.0 ug of untreated double-shelled virus. The position of molecular weight standards are indicated on the right hand side. The brackets ([) in Lane C indicate ladders formed in the 45K, 90K and 135K region.

FIGURE 18 shows the reactivity of antisera against the 232-256 VP3 synthetic peptide with the ladder complex on VP6 nucleocapsid monomers and dimers. Lane A represents the virus protein profile. Lane B represents the virus protein profile after complexing with 100 ug of the synthetic peptide and then treatment with .96 ug of trypsin; Lane B' represents the virus profile in Lane B electroblotted and reacted with anti-synthetic peptide antibodies. Lane C represents the virus protein profile after complexing with 100 ug of the synthetic peptide; Lane C' represents the virus profile in Lane C electroblotted and reacted with anti-synthetic peptide antibodies. The right hand side illustrated the location of molecular weight markers.

FIGURE 19 shows the investigation of sample buffer conditions necessary to maintain the 232-256 VP3 synthetic peptide-VP6 complex. Two micrograms of radiolabelled double-shelled rotavirus was reacted with 100 ug synthetic peptide for 30 min at 37° C. Prior to electrophoresis sample was aliquoted and treated with urea sample buffer, Lane A'; Laemmli sample buffer without BME, Lane B; and Laemmli sample buffer containing BME and boiled, Lane C. The arrowheads indicate the position of the 45K, 90K, 135 ladders. The position of the molecular weight standards are located on the right hand side.

FIGURE 20 shows the trypsin cleavage of the synthetic peptide. Lanes B, D and F represent trypsin at 19.2 ug, 9.6 ug and .96 ug respectively. Lane A, C and E represent the reaction of 100 ug of synthetic peptide with 19.2 ug, 9.6 ug and .96 ug of trypsin, respectively. Lane G represents 100 ug of the peptide with the arrows indicating the position of the monomer and dimer. Lane H indicates the position of molecular weight markers.

FIGURE 21 shows the competition of 232-256 VP3 synthetic peptide with the intact 84,000 VP3 for trypsin. Lane A represents the protein profile of double-shelled rotavirus. Lane C-F represents the viral protein profile after incubation of virus for 30 min at 37° C with .0097 ug of trypsin and increasing amounts of synthetic peptide. Lane B represents no synthetic peptide, Lane C has 25 ug; Lane D, 50 ug; Lane E, 75 ug; and Lane F, 200 ug of synthetic peptide. The positioned molecular weight markers are indicated on the left hand side. The arrowheads at Lane B denote the position of the doublet observed at 60,000 and the arrowhead at Lane F denotes the position of the 84,000 protein.

FIGURE 22 shows the effect of increasing amounts of synthetic peptide on the infectivity of rotavirus. Well A represents MA-104 cell control, well B represents the virus control approximately 100 PFU. Wells C-H represent duplicate samples of 100 PFU adsorbed to MA-104 cell monolayers in the presence of 100 ug, 200 ug and 300 ug of synthetic peptide, respectively.

## BACKGROUND OF THE INVENTION

Rotaviruses are important causes of gastrointestinal disorders and diarrhea in a wide variety of avian and animal species including man. The rotavirus genome consists of eleven segments of double-stranded RNA. These eleven genes encode the production of at least six structural proteins of the virus. In complete virus particles, these six proteins occur in a double-shelled arrangement. There are three inner shell proteins designated virus protein (VP) 1, 2 and 6. There are three outer shell proteins, two of which are designated VP3 and VP7. The third outer capsid protein, which is encoded by genomic segment 10 or 11, has not yet been assigned a number.

The molecular weights of these proteins are shown in Table 1.

Table 1

| Gene Assignment and Molecular Weight of the Major Rotavirus Structural Proteins | | | |
|---|---|---|---|
| Genomic Segment | Protein Designation | Molecular Weight[a] | Location[b] |
| 1 | VP1 | 110K | inner |
| 2 | VP2 | 92K | inner |
| 4 | VP3 | 84K | outer |
| 6 | VP6 | 45K | inner |
| 7 | | | |
| 8 triplet | VP7 | 41K | outer |
| 9 | | | |
| 10 or 11 | ND | 20K | outer |

a In different rotaviruses, the absolute order of the genomic segments does not always correspond to the same genes. For example, the electrophoretic order of segments 7, 8 and 9 changes among rotaviruses from different animal species. This is referred to as inversion or "flip-flopping" of genome segments. The gene triplet formed by segments 7, 8 and 9 codes for three polypeptides, the neutralization-specific major outer capsid glycoprotein identified as virus protein (VP) 7, and two nonstructural proteins which are not shown in this table. In rotavirus strains SA-11, W and Wa, gene 9 codes for VP7. In contrast, in rotavirus strain DS-1 and U.K. bovine rotavirus, gene 8 codes for VP7. There are discrepancies in the literature about the exact molecular weight of VP7, as well as of other rotavirus proteins. Several researchers have suggested this is in part due to the many variations in methods used to: 1) separate the individual polypeptides, 2) prepare virus samples for electrophoresis, 3) detect polypeptides in polyacrylamide gels and 4) detect various post-translational modifications of primary gene products. In addition, especially for bovine and human rotavirus, there are variations in the mobility of proteins derived from different isolates originating from the same species. The molecular weights shown in Table 1 are those reported by Sabara et al (Journal of Virology, Vol 53:58-66, 1985).

b Designates location of the structural protein in the inner or outer capsid of complete rotavirus particles.

Peptide subunits of VP7, VP6 and VP3 are the topic of this invention.

### a) Virus Protein 7 (VP7)

The major outer shell polypeptide, VP7, is a glycoprotein with an approximate molecular weight of 38,200 (38.2 K) in its unreduced form and 41,900 (41.9 K) in its reduced form. It has been shown to be the major antigen responsible for inducing neutralizing antibodies to the virus. This glycoprotein is also responsible for virus attachment to cells.

Different serotypes of rotavirus occur and are defined by the neutralizing activity stimulated by VP7. To date, seven serotypes have been identified; four of these (serotypes 1 to 4) are found in humans and five (serotypes 3 to 7) are found in animals. The importance of these serotypic differences is unclear because recent studies showed that in both animals and man, cross-protection among strains belonging to different serotypes may occur. This cross-protection may occur because there are common antigenic determinants of VP7 which are independent of serotype. Alternatively, the specific amino acid sequences within VP7 (epitopes) responsible for serotype specificity may induce some cross-reactive antibody that is responsible for cross-protection.

Having a molecular weight of 38.2/41.9 K, VP7 is made up of approximately 325 amino acids (Figure 1). The sequence of amino acids comprising VP7 of several different rotavirus isolates has been determined and indicates that the degree of amino acid homology ranges from 75 to 86%. Comparison of the sequences of these VP7's reveal several regions in which the amino acid sequence varies. However, prior to this disclosure, none of these fragments, nor any other similar natural or synthetic material has been used to demonstrate in vivo protective effects.

Epitope mapping of VP7 using neutralizing monoclonal antibodies localized a neutralizing-absorption domain to a component peptide with an approximate molecular weight of 14,000 (14 K). When purified, this 14 K peptide stimulated the formation of neutralizing antibodies in mice. The secondary structure of this peptide, as determined by disulphide bridges, is necessary for maintaining antigenicity. Within this 14 K peptide, four epitopes were identified which possessed some of the biological activity of VP 7 and of complete rotavirus particles.

Gunn et al (J.Virology (1985)54,791-797) discloses rotavirus VP7 amino acid sequences. Six peptides derived from such sequences were used to raise antibodies, although none of the antisera obtained either recognised whole virus or exhibited neutralizing activity.

b) Virus Protein 6 (VP6)

The 45K molecular weight nucleocapsid protein, although not an integral component of the outer capsid, is also an important antigen. It has been identified as the subgroup antigen by using several techniques including complement fixation, ELISA, immuno-adherence agglutination assay and specific monoclonal antibodies. The 45K nucleocapsid is also described as the common rotavirus group antigen since some monoclonal antibodies react with all rotaviruses and polyclonal serum raised against a single rotavirus type can detect most other rotavirus strains. Aside from its antigenic properties, the nucleocapsid protein is very immunogenic and several investigators have found that polyclonal serum raised to this protein has neutralizing ability.

Within bovine rotavirus, VP6 appears to exist in trimeric units in both the virus particle and in infected cells, with the intersubunit linkage consisting of noncovalent interactions. These trimeric units complex further by virtue of disulfide bridges into larger units which likely represent the ring-like structures observed by several investigators using electron microscopy. By employing different sample buffers, these nucleocapsid complexes can be visualized on polyacrylamide gels.

Four VP6-specific monoclonal antibodies with low neutralizing ability were produced. They reacted with the monomeric and trimeric units indicating that a site responsible for neutralization was exposed. Taking into account the configuration of the nucleocapsid in the virus particle, such a site can potentially be expressed at least eighteen times, in a tandem fashion, per ring-like structure. Further analysis of radiolabelled virus revealed that a high percentage (80%) of the infectious virus particles were partially double-shelled to the extent that monoclonal antibodies could recognize the VP6 and immunoprecipitate the virus particles. However, only a small percentage of these antibody-bound infectious virions could be neutralized by the antibody. These results may help to explain the high immunogenicity but low neutralizing ability of VP6.

The VP6-specific monoclonal antibodies also cross-reacted with simian (SA-11), pig (OSU), bovine (UK and NCDV), rhesus (RRV), and human (Wa and ST4) rotavirus isolates. Since these viruses belong to different sub-groups, it is likely that the monoclonal antibodies are recognizing a common antigenic site.

The published amino acid analysis of the VP6 of bovine, simian and human rotaviruses were employed, along with proteolytic digests and chemical cleavage of the bovine rotavirus protein, in order to identify the antigenic site recognized by our monoclonal antibodies. The intact VP6 is 397 amino acids in length (Figure 2). The smallest antibody-reactive fragment of VP6 had a molecular weight of approximately 6,300 (6.3K) and was comprised of 57 amino acids at positions 40 to 97. The results further indicated that within the 6.3K fragment, the putative subgroup-specific site lies between amino acids at positions 40 to 60.

c) Virus Protein 3 (VP3)

The outer shell polypeptide of rotavirus, VP3, is a protein comprised of 776 amino acids with an approximate molecular weight of 82,000 (82K) in its unreduced form and 84,000 (84K) in its reduced form (Figure 3). It has also been shown to be a major antigen responsible for inducing neutralizing antibodies to the virus. This protein also has the ability to hemagglutinate red blood cells and is responsible, in combination with VP7, in determining virus serotype.

The initial steps in rotavirus replication involve two major events; attachment of the virus particle to target cells and the penetration/uncoating of the virus particle within the cell. The enzyme trypsin enhances virus infectivity. However, this enhancement appears to act after adsorption, since trypsin does not affect the efficiency or rate of virus attachment to cells but does increase the levels of uncoated particles found in cells. The molecular mechanism for trypsin enhanced infectivity occurs via the cleavage of the VP3 protein which has a molecular weight of approximately 84,000, into two fragments with approximate molecular weights of 28,000 and 60,000. Therefore, the trypsin cleavage site of VP3 is important in rotavirus replication.

The 776 amino acids making up VP3 of bovine rotavirus have been sequenced. A partial amino acid sequence of simian VP3 has also been determined (Figure 3). Comparison of the amino acid sequences of these VP3 proteins illustrates that the peptide fragment mimicking the trypsin-cleavage site (box in Figure 3) is conserved between these two isolates of the virus. It is likely that this region is conserved among most of the rotaviruses since most rotaviruses require trypsin to enhance their infectivity.

We have synthesized the peptide corresponding to the trypsin cleavage site shown in Figure 3. The synthetic peptide exhibits three significant characteristics.

First, it stimulates the formation in animals of antibodies which neutralize the virus.

Second, it is able to bind to bovine rotavirus via binding to VP6 monomeric and oligomeric protein units. A practical application of this type of binding can be foreseen with respect to using the VP6 protein as a carrier in order to enhance immune responses to this synthetic peptide and perhaps to other synthetic peptides which contain the consensus binding structure. Since the interaction between the VP6 protein and the peptide is able to withstand harsh treatments, such as boiling in sodium dodecyl sulfate, it may be suitably stable in vaccine preparations. Also, using a carrier which has been shown to be an antigenic and highly immunogenic rotavirus protein increases the chances that better protection against disease can be achieved by using less of the immunogen in a vaccine preparation.

The third important feature of the synthetic peptide is that it can be cleaved by trypsin thereby demonstrating that it behaves like the authentic VP3 protein in this respect. Since the synthetic peptide can effectively compete with the natural 84,000 protein for trypsin, the rate of infection can be inhibited to some extent. Due to this fact, the synthetic peptide may also be valuable as a therapeutic agent against neonatal gastroenteritis.

In this specification, as is conventional in the art, an amino acid position in a given sequence is assigned a number equating to the number of amino acid residues in the sequence, counting from the N terminal to that position. In considering synthetic sequences, analogous to a natural polypeptide or to part of a natural polypeptide, it is generally convenient to base the numbering system on the natural polypeptide and to use that system for numbering the synthetic sequence, even though the synthetic sequence may be different from the natural sequence.

In other systems, short synthetic peptides corresponding to fragments of particular virus proteins have been shown to carry antigenic properties of the intact protein. Thus, synthetic antigens have been used for provoking antibodies against a variety of viruses including influenza, polio encephalomyelitis, foot and mouth disease and hepatitis B. We have now demonstrated similar properties for synthetic peptides of rotaviruses.

## SUMMARY OF THE INVENTION

The present invention is based on our discovery that peptides of rotavirus VP7, VP6 and VP3 possess important biological activities. The 14 K peptide of VP7, and shorter peptides within it comprising neutralizing amino acid sequences (epitopes), demonstrate at least a substantial portion of the immunogenic activity of the entire rotavirus glycoprotein or the rotavirus itself. Similarly, a 6.3K fragment of VP6 and shorter peptides within it also have neutralizing properties. The invention further relates to a synthetic peptide of VP3 which corresponds to the trypsin cleavage site of that protein and which has three important characteristics: 1) it stimulates the formation of neutralizing antibodies to the virus; 2) it binds to the virus particle via the VP6 protein; and 3) it mimics the trypsin-cleavage site in that it can be cleaved by trypsin and therefore can compete with virus for trypsin, thereby interfering with infectivity.

The short peptides (epitopes) can be synthesized directly or produced as fusion proteins, whereas the 14 K and 6.3 K peptides can be produced only as fusion proteins. Being produced as heterologous proteins, the peptides are essentially free of other proteins of viral origin and of the infectious agents itself and, therefore, can be used as vaccines without fear that the disease may be transmitted. Furthermore, these peptides are free of other protein material of mammalian origin.

The present invention, therefore, comprises immunogenic fusion polypeptides and synthetic peptides having amino acid sequences which correspond to at least a portion of the sequence of rotavirus VP7, VP6, and VP3. All of these peptides display a degree of immunogenicity sufficient to stimulate production of neutralizing rotavirus antibodies. Therefore, the invention is directed to compositions comprising the peptides and particularly compositions useful as vaccines and to the use of such vaccines to immunize avian and mammalian species against rotavirus infection. Furthermore, the peptide of VP3 can compete with the virus for trypsin and in this way interfere with virus infectivity thus acting as a therapeutic agent.

The first step is the production, by synthetic or recombinant DNA methods, of peptides corresponding to antigenic fragments of rotavirus VP7, VP6, or VP3. The peptides are then attached to suitable carriers and the resulting products are used as vaccines. Vaccination leads to the production of neutralizing rotavirus antibodies and the protection against infection.

Peptides having the following amino acid sequences stimulate the formation of rotavirus neutralizing antibody.

a) Virus Protein 7 (VP7)

Peptide 165-295 The amino acid sequence of the 14 K fragment of bovine rotavirus is shown in the box in Figure 1. This 14,000 molecular weight (14 K) fragment, spanning amino acids 165 to 295, was found to be responsible for much of the immunological activity of intact VP7 and was shown to be capable of eliciting antibodies which neutralized the virus and blocked viral attachment to host cells. Due to its large size (130 amino acid residues), the 14 K fragment comprises a plurality of antigenic determinants, several of which are in regions that undergo sequence changes or variability in different strains of rotavirus (variable strain-specific regions) and others in regions which are common or highly conserved among the various strains (conserved regions). Peptides corresponding to both types of regions can be used for the preparation of vaccines and include the following.

Peptide 175-183 Tyr-Gln-Gln-Thr-Asp-Glu-Ala-Asn-Lys

Peptide (179-183) - (251-259) Asp-Glu-Ala-Asn-Lys-Lys-Leu-Gly-Pro-Arg-Glu-Asn-Val-Ala

Peptides 175-183 and (179-183)-(251-259) do not belong to the invention.

**Peptide 247-259** Arg-Asn-Cys-Lys-Lys-Leu-Gly-Pro-Arg-Glu-Asn-Val-Ala

**Peptide 275-295** Pro-Thr-Thr-Ala-Pro-Gln-Thr-Glu-Arg-Met-Met-Arg-Ile-Asn-Trp-Lys-Lys-Trp-Trp-Gln-Val

b) Virus Protein 6 (VP6)

**Peptide 40-98** Thr-Met-Asn-Gly-Asn-Glu-Phe-Gln-Thr-Gly-Gly-Ile-Gly-Asn-Leu-Pro-Ile-Arg-Asn-Trp-Asn-Phe-Asn-Phe-Gly-Leu-Leu-Gly-Thr-Thr-Leu-Leu-Asn-Leu-Asp-Ala-Asn-Tyr-Val-Glu-Thr-Ala-Arg-Asn-Thr-Ile-Asp-Tyr-Phe-Val-Asp-Phe-Val-Asp-Asn-Val-Cys-Met.

This 6,300 molecular weight (6.3K) fragment spanning amino acids 40 to 97 (shown in the box in Figure 2) reacted with monoclonal antibodies which immunoprecipitated and neutralized the intact virus. Within this span of 57 amino acids, a shorter sequence also stimulates the formation of neutralizing antibodies and hence is useful as a vaccine. The amino acid sequence of this shorter peptide is as follows:

Peptide 40-60 Thr-Met-Asn-Gly-Asn-Glu-Phe-Gln-Thr-Gly-Gly-Ile-Gly-Asn-Leu-Pro-Ile-Arg-Asn-Trp-Asn.

c) Virus Protein 3 (VP3)

Peptide 232-256 Asn-Ile-Ala-Pro-Ala-Ser-Ile-Val-Ser-Arg-Asn-Ile-Val-Tyr-Thr-Arg-Ala-Gln-Pro-Asn-Gln-Asp-Ile-Ala

This peptide spans the trypsin cleavage site of VP3. Its location within the amino acid sequence of VP3 is shown in the box in Figure 3.

The 14K and 6.3K polypeptides and their component peptides of VP7 and VP6 respectively, and the peptide corresponding to the trypsin cleavage site of VP3, of this invention may be used for prevention and control, or diagnosis of rotavirus infections of birds and animals including man. These peptides, and combinations and modifications of them, attached to carriers known in the art, are effective as vaccines for either passive or active immunization. Further, the peptide corresponding to the trypsin cleavage site of VP3 may serve as a therapeutic agent to block or interfere with virus infection.

In accordance with the invention, it was discovered that neutralizing antibodies in laboratory animals are induced by these peptides covalently linked to keyhole lympet hemocyanin. Other carriers known in the art can also be used to make the conjugate with the peptide sequences of the invention including any natural or synthetic carrier. The term "carrier" is a recognized term in the art and literature and sometimes is referred to as "coupler", "protein carrier" or "hapten carrier". Natural carriers used in accordance with the invention are known and are typically keyhole limpet hemocyanin (KLH), bovine serum albumin (BSA), ovalbumin (OVA), Beta-galactosidase or penicillinase. Synthetic carriers are typically multi-poly-DL-alanyl-poly-L-lysine and poly-L-lysine. In addition, it was discovered that the synthetic peptide corresponding to the trypsin cleavage site of VP3 could itself stick to VP6 in a variety of forms. Therefore, VP6 could also serve as a carrier for the synthetic peptide corresponding to the trypsin cleavage site of VP3. This leads to the additional use of incorporating the amino acid sequence of the trypsin cleavage site of VP3 into a variety of macromolecules and binding them via this site to VP6 thereby increasing the immunogenicity of the macromolecules.

Generally, the peptide sequences of the invention are covalently attached to the molecule of the larger compound or, especially where it and the larger compound are constructed together by recombinant DNA technology, the sequences of the invention may be linked in some other manner to the larger compound. For example, where the larger compound is a polypeptide, such as Beta-galactosidase or penicillinase, it may be interposed in the amino acid chain of that compound. Further, the peptides may be synthesized in multiple repeats by recombinant DNA technology to produce a polypeptide of repeating epitopes containing the immunogenic region.

The invention also encompasses biologically active compositions comprising the peptides (antigens) and an immunostimulant or adjuvant and wherein the peptides are administered with the immunostimulant. Complete Freund's adjuvant, aluminum hydroxide and liposomes are examples of such immunostimulants. Other natural and synthetic immunostimulants are well known in the art. The administration of antigen and adjuvant need not be concurrent; one may proceed the other, in part or all of it.

The peptides of the present invention can be formulated according to known methods to prepare pharmaceutically useful compositions, whereby the polypeptides hereof are combined in admixture with a pharmaceutically acceptable carrier vehicle. Suitable vehicles and their formulation are described in Remington's Pharmaceutical Science by E.W. Martin, which is hereby incorporated by reference. Such compositions will contain an effective amount of the polypeptide product hereof, together with a suitable amount of carrier vehicle in order to prepare pharmaceutically acceptable compositions suitable for effective administration to the host. One preferred mode of administration is parenteral. Suitable animal health formulations are prepared, as in the case of pharmaceutical compositions, *mutatis mutandis*.

The vaccines of the present invention, incorporating a suitable polypeptide or peptide produced as herein described, can be prepared according to known methods, wherein said polypeptide or peptide is combined in admixture with a suitable vehicle. Suitable vehicles include, for example, saline solutions, various known adjuvants or other additives recognized in the art for use in compositions applied to prevent viral infections. Such vaccines will contain an effective amount of the polypeptides or peptides hereof and a suitable amount of vehicle in order to prepare a vaccine useful for effective administration to the host. Attention is also directed to New Trends and Developments in Vaccines, editors: A. Voller and H. Friedman, University Park Press, Baltimore, 1970, which is hereby incorporated by reference for further background details on the preparation of vaccines.

The peptides can be prepared according to any conventional method of synthesizing peptides. Either solid phase processes or liquid phase processes may be employed. Such processes for synthesizing peptides are described in, for example, "The Peptides", volume 2 (1984), E. Gross and J. Meinhaffer Editors, Academic Press, New York, U.S.A.

Also, it is possible to clone a DNA sequence corresponding to these amino acid sequences into vectors known in the art, for example pBR 322, since this plasmid readily accepts short lengths of DNA, and growth in, for example E coli HB101 or other hosts known in the art, would allow expression of the poly-peptide, which could be further enhanced by inclusion of promoters in the DNA sequence and other published procedures. Therefore, the invention is further directed to replicable DNA expression vectors which contain gene sequences which encode for the polypeptides in expressible form. The invention is also directed to recombinant host cells such as microorganism, strains or cell lines transformed with expression vectors known in the art and to the cultures thereof.

It is clear that all of the sequences which are given herein are by way of example only and that other composition related to relevant antigenic determinants or sequences in which limited conservative amino acid changes are introduced, can also be used.

## DETAILED DESCRIPTION OF THE INVENTION

### a) The 14 K Polypeptide of VP7

To prepare the various antigens to be used for immunization, Bovine Rotavirus (isolate C486, subclone 13), was propagated in MA-104 cells and purified by centrifugation. One milligram of purified double-shelled virus was then fractionated on a 10% preparative polyacrylamide gel. The 38.2 K glycoprotein was localized in the gel by staining side strips of the gel with Coomassie blue. To extract the glycoprotein, gel strips were subjected to electroelution.

The 14 K polypeptide fragment was prepared by placing a gel strip containing the 38.2 K glycoprotein (VP7)into the well of a 5% stacking-20% resolving polyacrylamide gel. A 13 cm X 1 cm gel strip was routinely treated with papain (Calbiochem-Behring, San Diego, Ca.) at a concentration of 130 ug/cm of gel; since this gave the best preparation of 14 K polypeptide as illustrated in Figure 4 (lane B). Electrophoresis and in situ enzyme digestion of the 38.2 K glycoprotein was performed according to the specifications of Cleveland et al (Cleveland, D.W., S.G. Fisher, Kirschner and U.K. Laemmli. 1977. Peptide mapping by limited proteolysis in SDS and analysis of gel electrophoresis. J. Biol. Chem. 252: 1102-1106) and prestained molecular weight markers were used in order to visualize the time of maximum resolution between the 14.3 K and 18.4 K markers. Further localization of the 14 K polypeptide was achieved using the molecular weight markers. The peptide fragment was then electroeluted from the gel slices, and a protein determination performed. Confirmation of the authenticity and purity of the peptide was by examination of its profile on polyacrylamide gels (Figure 4, lane C) and by its reaction with monoclonal antibody from hybridoma 11D12-6 (Figure 4, lane D).

The electroeluted 14 K polypeptide was then lyophilized and a portion of the preparation was conjugated to bovine serum albumin (BSA) as follows. One milligram of peptide was first dissolved in 125 ul of 0.1 M PBS pH 7.4. The BSA solution was prepared by dissolving 1.25 mg BSA in 600 ul 0.1 M PBS pH 7.4 and to this were added dropwise 250 ul of a 2.5 M gluteraldehyde solution and the peptide solution consecutively over 15 minutes. The reaction mixture was gently agitated for 24 hours at room temperature and then dialyzed extensively against sterile distilled water. Lyophilization of the conjugated peptide yielded a pinkish powder which was stored in dessicant at -20 °C.

Once all the antigens were prepared, groups of ten mice (Charles River, Wilmington, MA) were immunized with either the BSA-unconjugated polypeptide, BSA-conjugated polypeptide, infectious double-shelled virus or purified VP7 according to the protocol outlined in Table 2. The quantities of antigen to be administered were determined on an equimolar basis. Antibody responses to the different antigens were characterized by ELISA and immuno-blot ELISA using bovine rotavirus (isolate C486 subclones 12 and 13) as the antigen and by serum neutralization assays.

As illustrated in Figure 5 (upper panel) there was a significant antibody response to all the antigens used. Noteworthy is the similarity in response between VP7 (38.2 K glycoprotein) and the 14 K polypeptide fragment. It also appears that conjugation of the 14 K polypeptide to a carrier was not necessary to induce a good antibody response. This may be due to the large size of the polypeptide fragment thereby increasing the probability of it containing both B-cell and T-cell determinants.

The animals immunized with the 14 K fragments were boosted at 61 days with infectious, double-shelled virus. This was done in order to investigate the possibility of the fragments priming an immune response. Even though, after analysis of all the sera, the animals did show a good antibody response to the peptides, they also demonstrated an additional, albeit minor, response after the infectious virus was administered (68 days). These results were encouraging since they illustrated that the 14 K polypeptide alone was capable of inducing a respectable antibody titer. More importantly, these antibodies had neutralizing ability. Sera from groups A-D possessed neutralizing antibodies (Figure 5, lower panel), with the best response produced by animals immunized with infectious virus (Group D). Total antibody titers as measured by ELISA and neutralizing antibody titers were similar for both the conjugated and unconjugated form of the polypeptide. At 68 days, after all the groups had been exposed to infectious virus, the neutralizing antibody titer increased slightly over that seen at 51 days suggesting that each subsequent exposure further stimulates the immune response, or alternatively, there may be other antigens on the infectious virus that are capable of inducing a neutralizing response. The most likely candidates for such antigens are the minor outer shell protein (VP3, 84 K mol. wt.) and the major inner shell protein VP6 (45 K mol. wt.). Several reports have indicated that both of these proteins are capable of inducing neutralizing antibodies, although to a much lesser extent than the major glycoprotein. This, in fact, is supported by the presence of antibodies to the 45 K protein as illustrated by the immuno-blot ELISA reactions at 68 days even though antibodies to the 84 K protein could not be detected (Figure 6). As will be discussed further below monospecific and monoclonal antibodies to the 45 K antigen have demonstrated neutralizing ability.

Immuno-blot ELISA reactions of sera from selected animals in each group at 37, 51 and 68 days are shown in Figure 6. All the sera, except those obtained prior to immunization and the negative control group (E), possessed antibodies to VP7. Anti-peptide antibodies, although produced to the 14 K polypeptide prepared from only one of the glycoprotein species present in bovine rotavirus isolate C486, reacted with both glycoprotein species present in the protein profile of the parent isolate C486. This was significant since electrophoretypic analysis of the genomic RNA from the two subclones of isolate C486 demonstrated a difference in mobility of the corresponding genes coding for this glycoprotein. However, it appears that despite this genetic heterogeneity, the 14 K polypeptide is to a large extent, conserved between the two glycoprotein species of BRV isolate C486 subclones 12 and 13. Another interesting observation was the similar intensity displayed by the reaction of the glycoprotein species with anti-peptide antibodies suggesting that the 14 K polypeptide may represent an immunodominant region of the glycoprotein.

## The Component Peptides Within The 14 K Polypeptide

In order to be able to synthesize appropriate peptides it was necessary to localize the 14 K molecular weight polypeptide fragment within VP7. The following characteristics of the 14 K polypeptide were taken into consideration to accomplish this localization. The 14 K peptide has i) a carbohydrate moiety, ii) extensive disulfide bridging, iii) relatively conserved region(s) among different rotavirus serotypes, iv) hydrophilic areas; and v) potential immunogenic regions. In addition, the cleavage patterns obtained by partial proteolysis of the glycoprotein using chymotrypsin, Staph. aureus V8 protease, papain and cyanogen bromide aided in locating the 14 K polypeptide fragment (Figure 7).

The amino acid sequence of Nebraska calf diarrhea virus (NCDV bovine rotavirus), which exhibits high nucleic acid homology with the C486 bovine rotavirus and is of the same serotype, was used to map the 14 K polypeptide fragment to the region spanning amino acids 165-295. A hydrophilicity plot of the corresponding NCDV glycoprotein identified several hydrophilic regions within this area. Based on this, four peptides, corresponding to amino acid residues 174-183, 178-181 and 251-259 spliced together, 247-259, and 275-295 on VP7 of bovine rotavirus were synthesized by the solid phase peptide synthesis method of Merrifield.

The specific amino acid sequence of each peptide is as follows.

Peptide 175-183 Try-Gln-Gln-Thr-Asp-Glu-Ala-Asn-Lys

Peptide (179-183) - (251-259) Asp-Glu-Ala-Asn-Lys-Lys-Leu-Gly-Pro-Arg-Glu-Asn-Val-Ala

Peptide 247-259 Arg-Asn-Cys-Lys-Lys-Leu-Gly-Pro-Arg-Glu-Asn-Val-Ala

Peptide 275-295 Pro-Thr-Thr-Ala-Pro-Gln-Thr-Glu-Arg-Met-Met-Arg-Ile-Asn-Trp-Lys-Lys-Trp-Trp-Gln-Val

The purity of each peptide was assessed using thin layer chromatography and reverse phase high performance liquid chromatography. Fast atom bombardment mass spectromatry was used to confirm molecular weights.

The reactivity and specificity of the synthetic peptides was determined by several methods.

1) ELISA with anti-VP7 monospecific serum, indicating specificity of the peptides for VP7 (Table 3).

2) ELISA with monoclonal antibodies specific for the neutralizing glycoprotein (VP7) and which had the ability to block virus attachment, indicating specificity of the peptides for different regions or epitopes of VP7 (Table 3).

3) Adsorption blocking assay indicating that the two peptides, 247-259 and 275-295, blocked virus attachment to MA-104 cells, whereas the other two peptides did not (Figure 8).

The immunogenicity of the synthetic peptides was demonstrated in mice as follows.

Peptide 175-183 was conjugated to keyhole limpet hemocyanin (KLH) via a bis-diazotised tolidine linkage that produces N-terminally bound peptides. The other three peptides were conjugated to KLH via N-maleimidobenzoyl-N'-hydroxysuccinimide ester producing N-terminally bound peptide conjugates. Each peptide was administered to groups of 10 CD-1 mice according to the schedule outlined in Table 4.

Each mouse in groups 1 to 4 was given 100 ug of each KLH-conjugated peptide in Freund's Adjuvant. Group 5 was given 25 ug of each of the four KLH-conjugated peptides in Freund's Adjuvant. Group 6 received 1.6 ug of infectious double-shelled rotavirus and Group 7 represented the negative control group which received saline plus Freund's Adjuvant. The antigen preparations were administered three times over a six-week period and mice were bled prior to each immunization.

The reactivity and specificity of the antibodies elicited in mice by the polypeptides were reactive with complete rotavirus particles and individual corresponding peptides. Antibody titers to double-shelled rotavirus (upper panel - Figure 9) and to individual, corresponding peptides (lower panel - Figure 9) were determined by an enzyme-linked immunosorbent assay. All four synthetic peptides induced antibodies which reacted with rotavirus after the first immunization. Subsequent immunizations resulted in an increased response in all groups. Anti-peptide antibodies to the individual corresponding peptides were also induced after the first immunization indicating that all KLH-peptides were immunogenic (lower panel).

The mouse antibodies against two of the peptides were also capable of inhibiting the infectivity of the virus, as determined in vitro in plaque reduction assays performed as follows.

Neutralization of bovine rotavirus isolate C486 by mouse antisera was determined by a standard 50% plaque reduction assay. Virus dilutions representing 30-50 PFU were mixed 1:1 with various dilutions of antibody and incubated for 1 h at 37° C. Virus adsorption to MA-104 monolayers was allowed to proceed at 37° C for 2 h before the virus inoculum was removed; the cells were washed with MEN and then overlaid with 1.6% Bacto-agar (Difco) diluted in MEM and supplemented with 5 ug of pancreatin per ml, 0.7% of a 1:1,000 neutral red stock solution, and 0.1% DEAE-dextran. Plaques appeared after 5 to 6 days of incubation at 37° C.

As is shown in Figure 10, peptide 247-259, peptide 275-295 and the mixture of the four peptides induced virus neutralizing antibodies which increased after each immunization. This indicates that antibodies produced by these two polypeptides react with viral epitopes involved in virus attachment.

To demonstrate protection, a passive antibody transfer experiment was carried out using monoclonal antibody IOD2-7 which specifically recognizes synthetic peptide 275-295 (Table 5). Four groups each consisting of 10, 7 day old mice were first separated from their mothers for 2 hours and then given the appropriate Time 0 preparations by tubing to the stomach. Approximately 1 hour later they were given the Time 1 preparation. The mice were kept at 33° C for 8 hours with constant monitoring of fecal consistency. After 8 hours, the mice were sacrificed and their intestines removed and pulverized. The amount of infectious virus in the intestine was determined by 50% plaque-reduction assay.

As shown in Table 5, mice in Group III which did not receive monoclonal antibody were not protected. They became diarrheic and had 5 logs of rotavirus in intestinal homogenates prepared 8 hours after challenge. There was a significant reduction in the amount of diarrhea and in the level of virus in intestinal homogenates of mice in Group I (given monoclonal antibody orally before challenge with virus) and Group II (challenged with a mixture of monoclonal antibody and virus).

Further evidence of the immunogenicity and protective capacity of these VP7 peptides is presented below after the section b) on VP6.

b) The 6.3K. Polypeptide of VP6

Four monoclonal antibodies to the bovine rotavirus (isolate C486) VP6 (45K nucleocapsid protein) were identified via immunoprecipitation of infected cell lysates (Figure 11) and by an immuno-blot ELISA (Figure 12). The four monoclonal antibodies (1D7, 1B4, 1B9, 1DIO) demonstrated neutralizing ability similar to that observed for monospecific antiserum VP6, although lower than that exhibited by antisera to the two outer capsid proteins VP7 and VP3 (Table 6). In addition, a mixture of these four monoclonal antibodies recognized the nucleocapsid protein of a porcine (OSU), bovine (NCDV, UK) and monkey (SAII, RRV) rotaviruses belonging to sub-group 1, and human (WA, ST4) rotaviruses belonging to sub-group 2 in an immunoblot-ELISA (Figure 13).

In order to localize the antigenic determinants recognized by these monoclonal antibodies, immuno-blot ELISA reactions were carried out on nitrocellulose-blotted partial protein digests of VP6 using monoclonal antibodies and monospecific antiserum (Figure 14). Examination of the digests of each specific enzyme revealed that all four monoclonal antibodies recognized essentially the same peptides of VP6 in that digest. In the case of chymotrypsin and S. aureus V8 protease digests, the reactivity of monospecific serum was identical with that demonstrated by the monoclonal antibodies. Figure 15 illustrates the digestion pattern and antibody reactivity pattern of VP6 generated by chemical cleavage with cyanogen bromide (CNB4) and carboxypeptidase. As indicated by the arrows, the smallest antibody-reactive CNBr generated fragment had a molecular weight of approximately 6,300. Computer analysis of gene 6 translated sequence (Figure 2; Taken from Estes, M.K., B.B. Mason, S. Crawford and J. Cohen, 1984. Cloning and nucleotide sequence of the simian rotavirus gene 6 that codes for the major inner capsid protein. Nucelic Acids Research 12:1875-1887) indicated that the only two possible cyanogen bromide fragments that could react with our monoclonal antibodies are: from amino acids 40-97, molecular weight 6,302.7; and from amino acids 300-365, molecular weight 6,830 (Table 7). Antibody reactivity with the carboxypeptidase digest of VP6 indicated that the antigenic site is located at the amino terminal end of the molecule within the first 79 amino acids since a 9,500 molecular weight fragment was immune reactive, whereas a 6,000 molecular weight fragment (50 amino acids) was not (Figure 15, lane 1). Hence, the 6.3K polypeptide fragment responsible for the neutralizing activity is comprised of the 57 amino acids in position 40 to 97. These are enclosed in the box in Figure 2.

The Component Peptides of 6.3K of VP6

Using similar methods to those described above for the peptide fragments of the 14K polypeptide of VP7, the amino acid residue 40 to 60 of the 6.3K fragment of VP6 was synthesized.

The specific amino acid sequence of the peptide is as follows:

**Peptide 40-60** Thr-Met-Asn-Gly-Asn-Glu-Phe-Gln-Thr-Gly-Gly-Ile-Gly-Asn-Leu-Pro-Ile-Arg-Asn-Trp-Asn-Gly-Cys-OH

The terminal Gly-Cys-OH structure is not part of the antigenic fragment but was added to our sequence to provide a linkage site.

The reactivity and specificity of the synthetic polypeptide was determined by several methods.

1) ELISA with anti-VP6 monospecific serum, indicating specificity of the peptide for VP6 (Table 8).

2) ELISA with monoclonal antibodies specific for the neutralizing protein (VP6), indicating specificity of the peptide for the epitope of VP6 (Table 8).

3) Immunoblot ELISA with monoclonal antibodies, indicating specificity of the peptide for the epitope of VP6.

The immunogenicity and protective capacity of the peptides of VP6 and VP7 are further illustrated by the following examples.

The peptides corresponding to 275-295 of VP7 and 40-60 of VP6 were synthesized using Fmoc protected amino acids and solid phase peptide synthesis (Cambridge Research Biologics, Cambridge, England; IAF Biochemical, Laval, Quebec, Canada).

The peptide-Keyhole Limpet Hemacyanin (KLH) conjugates were produced using N-maleimido-benzoyl-N'-hydroxysuccinimide (MBS) ester derivitized KLH and a cysteine added to the N terminal of the peptide (Cambridge Research Biologics, Cambridge, England) or water soluble carbodiimide. The peptides were also conjugated to the E coli pilin protein K99. Urea extracted and ammonium sulfate precipitated K99 was purified and the peptides conjugated to it using the carbodiimide method. This protocol yielded a conjugate with a peptide to K99 ratio of 3.5:1 as determined by ultraviolet spectroscopy and amino acid analysis and confirmed by gel electrophoresis.

Following synthesis of peptides and conjugates the products were assessed for immunoreactivity using an immunoblot ELISA assay developed with polyclonal anti-rotavirus serum.

The ability of the synthetic peptide 275-295 of VP7 coupled to a K99 carrier to boost antibody levels in cows prior to parturition was tested. As illustrated below both cows had antibodies to rotavirus prior to immunization, and these titers did not increase after vaccination with the K99 carrier protein alone. After one immunization with K99 coupled to the synthetic peptide administered in DDA - aluminum hydroxide gel (100 ug of conjugate) the antibody response was increased by 1.0 - 1.5 logs.

| Antibody Titers to Rotavirus as Determined by ELISA | | | |
|---|---|---|---|
| | Prebleed | After K99 Alone | After K99 Plus Synthetic Peptide 275-295 of VP7 |
| Cow 1 | 8,000 | 8,000 | 100,000[a] |
| Cow 2 | 10,000 | 10,000 | 100,000 |

In a second experiment mice were immunized with 100 ug of either the KLH-peptide or the K99-peptide conjugates in Freund's Complete Adjuvant and the anti-rotavirus immune responses were monitored. The amount of the conjugates used in the immunizations contained approximately equal quantities of each of the rotavirus peptides. No difference in the response of mice to the conjugates was seen over 3 immunizations (Table 9). Although the two carriers differ greatly in molecular weight, KLH being approximately 3.5 x $10^6$ and K99 approximately 19,000 daltons, they appear to be equally effective in generating antibodies to these peptide haptens.

In addition to the use of carriers in producing immune responses to peptides, many adjuvants have been investigated to further increase these responses. Unfortunately, most adjuvants that are powerful enough to increase the immune responses are not acceptable for use in humans or animals. The adjuvant chosen for use in this study was the quartenary amino surfactant dimethyl dioctdecylammonium bromide (DDA). This adjuvant was selected because of its reported abilities as a powerful adjuvant toward haptens and the fact that it is approved for use in food animals. We used the K99-VP7 275-295 conjugate to compare effects of DDA and Freund's Complete Adjuvant.

Following immunization, both the anti-rotavirus titer and the anti-K99 activity found in the immunized mouse serum was measured. Each mouse received 100 ug of conjugate with either 0.1 ml FCA or 100 ug of DDA. Following two immunizations there was a significant increase in the rotavirus antibody titers in both groups (Table 10). The levels of rotavirus antibodies seen in this experiment approximate those seen following a protective immunization schedule using live rotavirus (Table 11).

We then examined the dose of antigen needed to produce this protective response. We used three doses of K99-VP7 peptide conjugate (1, 10 and 100 ug/mouse) combined with the adjuvant DDA. As shown in Table 11 the conjugate produced dose related responses to the carrier protein K99, but only the 100 ug dose approached the anti-rotavirus antibody level produced by virus-immunized controls.

In general "free" synthetic peptides generate only low titered, short duration responses. These responses have involved the use of strong adjuvants of limited practical usefulness. However, peptides which form aggregates, either spontaneously or through the use of lipid side chains, have produced functional titers. Alternatively, peptides have been used to prime the immune system and produce a secondary type of response when immunized with a suboptimal dose of parent protein. We have investigated several methods used to produce responses to other peptides. These methods include: 1) associating the peptides with liposomes containing an adjuvant, 2) attempting to aggregate the peptides with the adjuvant DDA, and 3) using peptides in combination with FCA to prime the immune response.

The data gathered from the previous studies have shown that synthetic peptides conjugated to carriers could produce levels of anti-rotavirus antibodies in the serum of mice that is similar to the titers seen following live virus inoculation. This encouraged us to use a rotavirus mousa model system for rotavirus diarrhea to demonstrate protection of neonates from disease challenge. Female mice were immunized three times during a schedule of breeding and pregnancy. The last immunization was given 2 weeks prior to whelping. Each of the carriers and synthetic peptides was used in combination with FCA. The K99-conjugates were also used with DDA (Table 12). The mouse pups were allowed to suckle and were challenged at 7 days of age with a bovine strain of rotavirus. The effectiveness of the immunizations was determined following challenge of the neonates in a single blind experiment. Morbidity, mortality and severity of diarrhea were scored over a 48 hr period following the challenge. Most diarrhea and morbidity was apparent within 3-5 hrs following challenge.

All synthetic peptide preparations provided a substantial reduction in morbidity, and two preparations provided protection equal to the whole virus. In this experiment K99 did not work as well as KLH as a carrier but peptide-K99 conjugates still provided protection to the challenge.

c) The Trypsin Cleavage Site of VP3

The amino acid sequence of the potential trypsin-cleavage site of VP3 was chosen based on the following facts: 1) the size of the fragments resulting from trypsin cleavage of the authentic VP3 protein are known, 2) the amino acid sequence of the authentic VP3 is known (Figure 3) and, 3) the cleavage specificity for the enzyme trypsin is known. The specific amino acid sequence of the cleavage site is as follows: Asn-Ile-Ala-Pro-Ala-Ser-Ile-Val-Ser-Arg-Asn-Ile-Val-Tyr-Thr-Arg-Ala-Gln-Pro-Asn-Gln-Asp-Ile-Ala. This peptide has a molecular weight of 2,753 and represents amino acids 232 to 256 of VP3.

Figure 16 illustrates that antisera to the synthetic peptide reacted specifically with both the reduced (denoted 1 and 1') and unreduced (denoted 4 and 4') VP3. Since it specifically reacted with the lower band of the closely migrating 84,000 doublet (denoted as 1) this unequivocally identifies it as the product of gene 4 and ensures that the synthetic peptide corresponded to this site on authentic VP3.

Since the trypsin cleavage site is a biologically important region it was important to determine whether antiserum to this peptide could neutralize virus infectivity. As illustrated in Table 13 below, antiserum to the synthetic peptide as well as monoclonal antibodies specifically reacting with the peptide were able to effectively neutralize virus infectivity.

Table 13

| Neutralizing Ability of Antisera to the 232-256 Synthetic Peptide of VP3 | |
|---|---|
| Antibody | Virus Neutralizing Titre Determined By 50% Plaque Reduction Assay |
| Antiserum to Synthetic Peptide | 5,000 |
| Monoclonal Antibody to Synthetic Peptide | 10,000 |

When 100 ug of the synthetic peptide was reacted with 2.0 ug of purified virus for 30 min at 37°C a laddering of VP6 (the 45K nucleocapsid protein) was observed (Figure 17, lane C). At lower concentrations of the peptide, i.e. 25 ug, the laddering was not obvious (lane A). A corresponding ladder to that at the 45K location was also observed at the 90K and 135K regions (lane C). Support for the binding of the peptide to VP6 was provided by the fact that the molecular weight increments in the ladder correspond to the molecular weight of the synthetic peptide monomer. In addition, trypsin-treatment of the virus-peptide complex (lanes B and D) reduced the ladder in both the nucleocapsid monomer (45K), dimer (90K) and trimer (135K) to the extent that the virus profile was almost identical to the uncomplexed, trypsin-treated virus profile (lane E).

Definitive proof that the peptide bound to VP6 was demonstrated by the fact that a ladder was detected in both the 45K and 90K region with antisera produced against the synthetic peptide (Figure 18, lane C). Reactivity was not observed however, when the virus was reacted with the synthetic peptide and then treated with 0.96 ug of trypsin (Figure 18, lane B) indicating that even when it is bound to the VP6 protein it maintains the trypsin-cleavage site.

Reactivity of the 232-256 VP3 synthetic peptide with VP6 was maintained under conditions where samples were treated with urea sample buffer for 30 min at 37°C (Figure 19, lane A) and when samples were treated with Laemmli buffer without B-mercaptoethanol (BME) but with boiling (lane B). However, when BME was included in the sample buffer and the sample was boiled, prior to electrophoresis, the ladders in both the 45K and 90K regions (indicated by arrowhead) disappeared (lane C), suggesting that secondary structure specified by disulphide bridging was necessary to maintain the VP6-synthetic peptide complex.

The synthetic peptide corresponding to the trypsin cleavage site of VP3 was also tested for its authenticity by reacting it with trypsin. Upon electrophoresis of the peptide, both monomeric and dimeric forms were observed after staining with Coomassie blue (Figure 20, lane G). The monomer, however, is not clearly visible since it quickly diffused out of the 17.5% resolving gel upon destaining. Treatment of the synthetic peptide with 9.6 ug (lane C) and 19.2 ug (lane A) of trypsin/100 ug of synthetic peptide for 30 min at 37° C reduced the amount of visible peptide by 90-100%. At a concentration of 0.96 ug of trypsin/100 ug of peptide, approximately 30% of the peptide remained uncleaved (lane E). The progressive cleavage of the peptide by increasing concentrations of trypsin indicates that the peptide is being specifically recognized by the enzyme at one of two potential sites.

Since we had established that the synthetic peptide could be recognized and cleaved by trypsin we investigated whether it could compete with the intact 84K VP3 for this enzyme. The extent of competition was measured by the ability of increasing concentrations of the synthetic peptide to reduce the amount of the authentic VP3 to be cleaved into its two products (molecular weights of approximately 60,000 indicated by lower arrowheads in lane B, and 28,000 indicated by upper arrowhead in lane F of Figure 21). The amount of trypsin used in this experiment was established such that the lowest quantity of synthetic peptide used, i.e. 25 ug of peptide and 25 ug of VP3 were completely cleaved (Figure 21, lane B). As the amount of synthetic peptide increased to 200 ug VP3 becomes evident (indicated by arrowhead in lane F), thereby attesting to the authenticity of the synthetic peptide.

Since the synthetic peptide could bind to the nucleocapsid protein and still be cleaved by trypsin we wanted to investigate whether it had any effect on virus infectivity. When increasing amounts of the synthetic peptide were mixed with infectious virus the number of visible plaques were reduced. At high concentrations (Figure 22, lanes E-H) of the synthetic peptide the plaques were only visible 5-6 days after plaques in the control well (B) were seen. These plaques however, were very small and diffuse and were not readily seen in wells E-H. Based on this information, the synthetic peptide may have a therapeutic, in addition to a prophylactic, application.

The dosage range for vaccination against rotavirus related diseases is from about 1-100 ug of peptide per kg of body weight. It has been found that this range provides an effective immune response for any of the peptides disclosed herein.

It is possible to replace one or more of the amino acid residues in the above sequences by other amino acid residues, which replacement can be expected to have no major effect on antigenicity or immunogenicity. It is also possible to add or delete amino acid residues within or flanking these sequences without dramatically altering antigenicity or immunogenicity. Thus, antigenic and immunogenic equivalents of these sequences also form part of the present invention.

Table 2

**Table 2 Schedule for Mice Immunized with 14k Peptide, VP7 and Infectious Virus**

| Groups/Immunogen | DAYS 1 | 8 | 31 | 37 | 44 | 51 | 61[2] | 68 |
|---|---|---|---|---|---|---|---|---|
| A. Unconjugated 14K | prebleed | 13.6ug; I.P.;F.C.[1] | 13.6ug; I.P.;F.I. | Bleed | 13.6ug; I.P.;F.I. | Bleed | 0.675ug; I.P.;F.I. | Bleed |
| B. Conjugated 14K | " | 13.6ug; I.P.;F.C. | 13.6ug; I.P.;F.I. | " | 13.6ug; I.P.;F.I. | " | 0.675ug; I.P.;F.I. | " |
| C. Glycoprotein VP7 | " | 4.5ug; I.P.;F.C. | 4.5ug; I.P.;F.I. | " | 4.5ug; I.P.;F.I. | " | 0.675ug; I.P.;F.I. | " |
| D. Infectious virus | " | 0.675ug; I.P.;F.C. | 0.675ug; I.P.;F.I. | " | 0.675ug; I.P.;F.I. | " | 0.675ug; I.P.;F.I. | " |
| E. Negative control | " | saline; I.P.;F.C. | saline; I.P.;F.I. | " | saline; I.P.;F.I. | " | $E_1$-0.675ug; I.P.;F.I.<br>$E_2$-saline; I.P.;F.I. | "<br>" |

The quantities given are per mouse (10 mice per group); I.P.-intraperitoneal; F.C.-Freund's complete adjuvant; F.I.-Freund's incomplete adjuvant

Table 3

**Table 3**

**Reactivity[a] of Monoclonal Antibodies and Monospecific Serum With Synthetic Peptides of the 14K Fragment of VP7**

| | SYNTHETIC PEPTIDES | | | |
|---|---|---|---|---|
| | p174-183 | p178-181-251-259 | p247-259 | p275-295 |
| Monospecific anti-glycoprotein (VP7) serum | 2,500[a] | 5,000 | 1,000 | 1,250 |
| Monoclonal antibodies | | | | |
| 4B5-5 | 50 | 50 | 5,000 | 50 |
| 11D10-4 | 10 | 250 | 4,000 | 100 |
| 11D12-6 | 10 | 50 | 100 | 8,500 |
| 10D2-7 | 10 | 50 | 20 | 10,000 |

[a] antibody titers were determined by ELISA and are expressed as the reciprocal of the dilution giving a 50% end point.

Table 4

**Table 4. Immunization Schedule for Mice Injected with Synthetic Peptides Within 14K of VP7**

| Group Designation | Immunization Given At Week: 0 | 1 | 2 | 3 | 4 | 5 | 6 | 7 |
|---|---|---|---|---|---|---|---|---|
| 1<br>p174-183 | Pre-bleed | -100ug; FC[a] | -- | -- | -bleed<br>-100ug; FI[a] | -- | -bleed<br>-100ug; FI | -bleed |
| 2<br>p(178-181)-(251-259) | Pre-bleed | -100ug; FC | -- | -- | -bleed<br>-100ug; FI | -- | -bleed<br>-100ug; FI | -bleed |
| 3<br>p247-259 | Pre-bleed | -100ug; FC | -- | -- | -bleed<br>-100ug; FI | -- | -bleed<br>-100ug; FI | -bleed |
| 4<br>p275-295 | Pre-bleed | -100ug; FC | -- | -- | -bleed<br>-100ug; FI | -- | -bleed<br>-100ug; FI | -bleed |
| 5<br>p174-183<br>p178-(181-251)-259<br>p247-259<br>p275-295 | Pre-bleed | -25ug;FC<br>-25ug;FC<br>-25ug;FC<br>-25ug;FC | -- | -- | -bleed<br>-25ug;FI<br>-25ug;FI<br>-25ug;FI<br>-25ug;FI | -- | -bleed<br>-25ug;FI<br>-25ug;FI<br>-25ug;FI<br>-25ug;FI | -bleed<br>-25ug;FI<br>-25ug;FI<br>-25ug;FI<br>-25ug;FI |
| 6<br>Infectious Virus | Pre-bleed | -1.6ug;FC | -- | -- | -bleed<br>-1.6ug;FI | -- | -bleed<br>-1.6ug;FI | -bleed<br>-1.6ug;FI |
| 7<br>Controls | Pre-bleed | -saline;FC | -- | -- | -bleed<br>-saline;FI | -- | -bleed<br>-saline;FI | -bleed<br>-saline;FI |

[a] FC = Freund's complete adjuvant; FI = Freund's incomplete adjuvant.

Table 5

**Table 5. Passive Antibody Transfer of Monoclonal Antibody 10D2-7**

| Group Designation | Time 0[a] Preparations | Time 1[a] Preparations | Titer[b] (PFU/ml) | Diarrhea[c] |
|---|---|---|---|---|
| I | 1:50 dilution of MAB 10D2-7 | $5 \times 10^6$ PFU/ml mouse rotavirus | $3.5 \times 10^2$ PFU/ml | $\pm$ |
| II | 1:50 dilution of MAB 10D2-7 was mixed with $5 \times 10^6$ PFU/ml of mouse rotavirus 1 hour prior to administration to neonates | MEM | $5 \times 10^1$ PFU/ml | - |
| III | MEM | $5 \times 10^6$ PFU/ml mouse rotavirus | $4 \times 10^5$ PFU/ml | + |
| IV | MEM | MEM | 0 | - |

a Time 0 and Time 1 preparations were made as described above. 100 ul of the preparations were administered by tubing to the stomach of each neonate.

b PFU/ml = plaque-forming units of rotavirus per ml of intestinal homogenate.

c Diarrhea was assessed by the color and consistency of the fecal material compared to the control groups (III and IV).

Table 6

**TABLE 6 Neutralizing Antibody Titers to Whole Rotaviruses of Polyclonal and Monoclonal Antibodies**

| Antibody Type | Neutralizing Titer |
|---|---|
| Polyclonal | |
| anti-45K (VP6) | 500[a] |
| anti-38.2K/41.9K (VP7) | 200,000 |
| anti-82K/84K (VP3) | 5,000 |
| Monoclonal to VP6 | |
| 1D7 | 500 |
| 1B4 | 500 |
| 1B9 | 800 |
| 1D10 | 500 |

a Neutralizing titre determined by the reciprocal of antibody dilution necessary to produce a 50% plaque reduction. The antibody concentration was standardized based on ELISA titers.

Table 7

**TABLE 7 Cyanogen Bromide Cleavage Fragments of VP6 Based on Translated Sequence of Estes et al.**

| Amino Acid Position From | To | Molecular Weight | No. of Residues |
|---|---|---|---|
| 180 | 295 | 12,989.170 | 115 |
| 100 | 177 | 9,085.530 | 77 |
| 300 | 365 | 6,830.448 | 65 |
| 41 | 97 | 6,302.700 | 56 |
| 394 | 444 | 4,944.510 | 50 |
| 1 | 37 | 4,133.350 | 36 |
| 365 | 394 | 3,414.630 | 29 |
| 295 | 300 | 613.710 | 5 |
| 37 | 41 | 476.610 | 4 |
| 97 | 100 | 393.400 | 3 |
| 177 | 180 | 307.360 | 3 |

Table 8

**Table 8. Reactivity of Monoclonal Antibodies and Monospecific Serum with Synthetic Peptide of VP6**

| | P-40-60 |
|---|---|
| Monospecific anti-VP6 serum | 10,000 |
| Monoclonal Antibodies | |
| 1D7 | 5,000 |
| 1B4 | 5,000 |
| 1B9 | 8,000 |
| 1D10 | 6,500 |

Table 9

**TABLE 9. THE EFFECT OF CARRIER ON THE RESPONSE TO VP7 275-295 PEPTIDE AND VP6 40-60 PEPTIDE**

| Peptide | Carrier | Anti-Rotavirus ELISA Titers Following Immunization[1]: 2 Weeks Post 1st Immunization | 2 Weeks Post 2nd Immunization | 2 Weeks Post 3rd Immunization |
|---|---|---|---|---|
| VP6 - 40-60 | KLH | 1,333 | 13,335 | 42,170 |
| | K99 | 13,335 | 31,623 | 74,990 |
| VP7 - 275-295 | KLH | 4,870 | 42,170 | 56,234 |
| | K99 | 7,500 | 23,713 | 56,234 |

[1] All mice were seronegative at the time of first immunization.

Table 10

TABLE 10. THE EFFECT OF ADJUVANT ON THE RESPONSE TO K99- VP7-275-295 CONJUGATE

| Adjuvant | | ELISA Titers Following Immunization[1] Two Weeks Post First Immunization | ELISA Titers Following Immunization[1] Two Weeks Post Second Immunization |
|---|---|---|---|
| Freund's Complete Adjuvant | Anti-rota | 9 | 6,310 |
| | Anti-K99 | 31,600 | 50,120 |
| Dimethyl dioctadecyl Ammonium Bromide | Anti-rota | 89 | 7,495 |
| | Anti-K99 | 251,200 | 10,000,000 |

[1] All mice were seronegative at the first immunization.

Table 11

**Table 11. EFFECT OF CONJUGATE DOSE ON THE ANTIBODY RESPONSE TO VP7 275-295 PEPTIDE AND TO THE K99 CARRIER PROTEINS**

| Dose of Conjugate | | ELISA Titer Following Immunizations[1] 2 Weeks Post First Immunization | 2 Weeks Post Second Immunization |
|---|---|---|---|
| 1 ug | Anti-rotavirus | 1.5 | 2.5 |
| | Anti-K99 | 63 | 4,500 |
| 10 ug | Anti-rotavirus | 1.5 | 2.5 |
| | Anti-K99 | 710 | 79,500 |
| 100 ug | Anti-rotavirus | 89 | 7,495 |
| | Anti-K99 | 251,200 | 10,000,000 |
| (Virus Control) | Anti-Rotavirus | | 15,850 |
| | Anti-K99 | | 0 |

[1] All mice were seronegative at the first immunization.

Table 12

Table 12. PROTECTION OF NEONATAL MICE FROM ROTAVIRUS CHALLENGE FOLLOWING 3 IMMUNIZATIONS OF DAMS WITH 100 UG OF IMMUNOGEN

| Immunogen | Adjuvant | Diarrhea Score |
|---|---|---|
| KLH | FCA[a] | 4+ |
| KLH (40-60) of VP6 | FCA | 1+ |
| KLH (275-295) of VP7 | FCA | - |
| K99 (40-60) of VP6 | FCA | 1+ |
| K99 (275-295) of VP7 | FCA | 2+ |
| K99 (40-60) of VP6 | DDA[b] | +/- |
| K99 (275-295) of VP7 | DDA | 2+ |
| Whole Virus Control | FCA | - |

Diarrhea Scored 4+ (Severe) to - (None)

[a] Freund's Complete Adjuvant

[b] Dimethyl Dioctyl Decyl Ammonium Bromide

## Claims

1. A peptide having an amino acid sequence corresponding to positions 165-295 of the intact 41 kd neutralizing glycoprotein (VP7) of rotavirus, or an antigenic equivalent thereof, said peptide having a molecular weight of 14 kd and the immunological activity of eliciting antibodies that neutralize intact rotavirus.

2. The peptide of claim 1 which has the amino acid sequence:

Cys-Asn-Pro-Met-Asp-Ile-Thr-Leu-Tyr-Tyr-Tyr-Gln-Gln
-Thr-Asp-Glu-Ala-Asn-Lys-Trp-Ile-Ser-Met-Gly-Ser-Ser-Cys-Thr
-Val-Lys-Val-Cys-Pro-Leu-Asn-Thr-Gln-Thr-Leu-Gly-Ile-Gly-Cys
-Leu-Ile-Thr-Asn-Pro-Asp-Thr-Phe-Glu-Thr-Val-Ala-Thr-Thr-Glu
-Lys-Leu-Val-Ile-Thr-Asp-Val-Val-Asp-Gly-Val-Asn-His-Lys-Leu
-Asn-Val-Thr-Thr-Ala-Thr-Cys-Thr-Ile-Arg-Asn-Cys-Lys-Lys-Leu
-Gly-Pro-Arg-Glu-Asn-Val-Ala-Ile-Ile-Gln-Val-Gly-Gly-Ala-Asn
-Val-Leu-Asp-Ile-Thr-Ala-Asp-Pro-Thr-Thr-Ala-Pro-Gln-Thr-Glu
-Arg-Met-Met-Arg-Ile-Asn-Trp-Lys-Lys-Trp-Trp-Gln-Val,

or
an antigenic equivalent thereof.

3. A peptide having an amino acid sequence corresponding to positions 40-97 of the nucleocapsid protein VP6 of rotavirus, or an antigenic equivalent thereof, said peptide having a molecular weight of 6.3 kd and the immunological activity of eliciting antibodies that neutralize intact rotavirus.

4. The peptide of claim 3 which has the amino acid sequence:

Thr-Met-Asn-Gly-Asn-Phe-Gln-Glu-Thr-Gly-Gly-Ile-
Gly-Asn-Leu-Pro-Ile-Arg-Asn-Trp-Asn-Phe-Asn-Phe-Gly-Leu-Leu-
Gly-Thr-Thr-Leu-Leu-Asn-Leu-Asp-Ala-Asn-Tyr-Val-Glu-Thr-Ala-
Arg-Asn-Thr-Ile-Asp-Tyr-Phe-Val-Asp-Phe-Val-Asp-Asn-Val-Cys-
Met;

or
an antigenic equivalent thereof.

5. A peptide having an amino acid sequence corresponding to positions 40-60 of the nucleocapsid protein VP6 of rotavirus or an antigenic equivalent thereof, said peptide having the immunological activity of eliciting antibodies that neutralize the intact rotavirus.

6. The peptide of claim 5 which has the amino acid sequence:

Thr-Met-Asn-Gly-Asn-Glu-Phe-Gln-Thr-Gly-Gly-Ile-
Gly-Asn-Leu-Pro-Ile-Arg-Asn-Trp-Asn;

or
an antigenic equivalent thereof.

7. A peptide having an amino acid sequence corresponding to positions 232-256 of the 84 kd neutralizing glycoprotein (VP3) of rotavirus, or the antigenic equivalent thereof, said peptide having immunological activity of eliciting antibodies that neutralize intact rotavirus.

8. The peptide of claim 7 which has the amino acid sequence:

Asn-Ile-Ala-Pro-Ala-Ser-Ile-Val-Ser-Arg-Asn-Ile-Val-Tyr-Thr-Arg-Ala-Gln-Pro-Asn-Gln-Asp-Ile-Ala,

or the sequence

Asn-Ile-Val-Pro-Val-Ser-Ile-Val-Ser-Arg-Asn-Ile-Val-Tyr-Thr-Arg-Ala-Gln-Pro-Asn-Gln-Asp-Ile-Ala,

Gln-Ile-Val-Pro-Val-Ser-Ile-Val-Ser-Arg-Gln-Ile-Val-Val-Tyr-Arg-Ala-Asn-Pro-Gln-Asn-Asp-Ile,

or the antigenic equivalents thereof.

9. A vaccine formulated for inoculation of a subject to be protected against rotavirus infection comprising an effective amount of a peptide or mixture of peptides as defined in claims 1-8, said vaccine being capable of eliciting antibodies that neutralize intact rotavirus.

10. The vaccine of claim 9 wherein the peptide is conjugated to a macromolecular carrier.

11. The vaccine of claim 10 wherein the carrier is selected from keyhole limpet hemocyanin (KLH), bovine serum albumin (BSA), ovalbumin (OVA), beta-galactosidase, beta-penicillinase, multipoly-DL-alanyl-poly-L-lysine, poly-L-lysine, and VP6 rotavirus protein.

12. A vaccine formulated for inoculation of a subject to be protected against rotavirus infection which comprises an effective amount of a peptide or mixture of peptides, said vaccine being capable of eliciting antibodies that neutralize intact rotavirus,

wherein said peptides are selected from a peptide having an amino acid sequence corresponding to positions 247-259 of intact VP7 or an antigenic equivalent thereof, and a peptide having an amino acid sequence corresponding to positions 275-295 of intact VP7 or an antigenic equivalent thereof.

13. The vaccine of claim 12 wherein said peptides have the amino acid sequence:

Arg-Asn-Cys-Lys-Lys-Leu-Gly-Pro-Arg-Glu-Asn-Val-Ala;

and

Pro-Thr-Thr-Ala-Pro-Gln-Thr-Glu-Arg-Met-Met-Arg-Ile-Asn-Trp-Lys-Lys-Trp-Trp-Gln-Val;

respectively.

14. The vaccine of claim 12 or claim 13 wherein the peptide is conjugated to a macromolecular carrier.

15. The vaccine of claim 14 wherein the carrier is selected from keyhole limpet hemocyanin (KLH), bovine serum albumin (BSA), ovalbumin (OVA), beta-galactosidase, beta-penicillinase, multipoly-DL-alanyl-poly-L-lysine, poly-L-lysine, and VP6 rotavirus protein.

16. A peptide according to any one of claims 1-8 or a vaccine according to any one of claims 9-15 for use in the treatment or prophylaxis of rotavirus infection in mammals or fowl.

**Revendications**

1. Peptide ayant une séquence d'acides aminés correspondant aux positions 165-295 de la glycoprotéine neutralisante intacte à 40 kd (VP7) de rotavirus, ou un de ses équivalents antigéniques, ledit peptide ayant un poids moléculaire de 14 kd et une activité immunologique d'induction de la formation d'anticorps qui neutralisent les rotavirus intacts.

2. Peptide de la revendication 1 qui a la séquence d'acides aminés:

Cys-Asn-Pro-Met-Asp-Ile-Thr-Leu-Tyr-Tyr-Tyr-Gln-Gln
-Thr-Asp-Glu-Ala-Asn-Lys-Trp-Ile-Ser-Met-Gly-Ser-Ser-Cys-Thr
-Val-Lys-Val-Cys-Pro-Leu-Asn-Thr-Gln-Thr-Leu-Gly-Ile-Gly-Cys
-Leu-Ile-Thr-Asn-Pro-Asp-Thr-Phe-Glu-Thr-Val-Ala-Thr-Thr-Glu
-Lys-Leu-Val-Ile-Thr-Asp-Val-Val-Asp-Gly-Val-Asn-His-Lys-Leu
-Asn-Val-Thr-Thr-Ala-Thr-Cys-Thr-Ile-Arg-Asn-Cys-Lys-Lys-Leu
-Gly-Pro-Arg-Glu-Asn-Val-Ala-Ile-Ile-Gln-Val-Gly-Gly-Ala-Asn
-Val-Leu-Asp-Ile-Thr-Ala-Asp-Pro-Thr-Thr-Ala-Pro-Gln-Thr-Glu
-Arg-Met-Met-Arg-Ile-Asn-Trp-Lys-Lys-Trp-Trp-Gln-Val,

ou un de ses équivalents antigéniques.

3. Peptide ayant une séquence d'acides aminés correspondant aux positions 40-97 de la protéine de nucléocapside VP6 de rotavirus, ou un de ses équivalents antigéniques, ledit peptide ayant un poids moléculaire de 6,3 kd et une activité immunologique d'induction de la formation d'anticorps qui neutralisent les rotavirus intacts.

4. Peptide de la revendication 3 qui a la séquence d'acides aminés:

Thr-Met-Asn-Gly-Asn-Phe-Gln-Glu-Thr-Gly-Gly-Ile-
Gly-Asn-Leu-Pro-Ile-Arg-Asn-Trp-Asn-Phe-Asn-Phe-Gly-Leu-Leu-
Gly-Thr-Thr-Leu-Leu-Asn-Leu-Asp-Ala-Asn-Tyr-Val-Glu-Thr-Ala-
Arg-Asn-Thr-Ile-Asp-Tyr-Phe-Val-Asp-Phe-Val-Asp-Asn-Val-Cys-
Met;

ou un de ses équivalents antigéniques.

5. Peptide ayant une séquence d'acides aminés correspondant aux positions 40-60 de la protéine de nucléocapside VP6 de rotavirus ou un de ses équivalents antigéniques, ledit peptide ayant une activité immunologique d'induction de la formation d'anticorps qui neutralisent les rotavirus intacts.

6. Peptide de la revendication 5 qui a la séquence d'acides aminés:

Thr-Met-Asn-Gly-Asn-Glu-Phe-Gln-Thr-Gly-Gly-Ile-Gly-Asn-Leu-Pro-Ile-Arg-Asn-Trp-Asn;

ou un de ses équivalents antigéniques.

7. Peptide ayant une séquence d'acides aminés correspondant aux positions 232-256 de la glycoprotéine neutralisante à 84 kd (VP3) de rotavirus, ou son équivalent antigénique, ledit peptide ayant une activité antigénique d'induction de la formation d'anticorps qui neutralisent les rotavirus intacts.

8. Peptide de la revendication 7 qui a la séquence d'acides aminés:

Asn-Ile-Ala-Pro-Ala-Ser-Ile-Val-Ser-Arg-Asn-Ile-Val-Tyr-Thr-Arg-Ala-Gln-Pro-Asn-Gln-Asp-Ile-Ala,

ou la séquence

Asn-Ile-Val-Pro-Val-Ser-Ile-Val-Ser-Arg-Asn-Ile-Val-Tyr-Thr-Arg-Ala-Gln-Pro-Asn-Gln-Asp-Ile-Ala,

ou leurs équivalents antigéniques.

9. Vaccin formulé pour inoculation d'un sujet à protéger contre l'infection à rotavirus comprenant une quantité efficace d'un peptide ou mélange de peptides tels que définis dans les revendications 1-8, ledit vaccin étant capable d'induire de la formation d'anticorps qui neutralisent les rotavirus intacts.

10. Vaccin de la revendication 9 dans lequel le peptide est conjugué à un support macromoléculaire.

11. Vaccin de la revendication 10 dans lequel le support est choisi parmi l'hémocyanine de patelle (KLH), la sérum-albumine de boeuf (SAB), l'ovalbumine (OVA), la bêta-galactosidase, la bêta-pénicillinase, la multipoly-DL-alanyl-poly-L-lysine, la poly-L-lysine et la protéine de rotavirus VP6.

12. Vaccin formulé pour inoculation d'un sujet à protéger contre l'infection à rotavirus qui comprend une quantité efficace d'un peptide ou mélange de peptides, ledit vaccin étant capable d'induire de la formation d'anticorps qui neutralisent les rotavirus intacts, où lesdits peptides sont choisis parmi un peptide ayant une séquence d'acides aminés correspondant aux positions 247-259 de VP7 intacte ou un de ses équivalents antigéniques, et un peptide ayant une séquence d'acides aminés correspondant aux positions 275-295 de VP7 intacte ou un de ses équivalents antigéniques.

13. Vaccin de la revendication 12 dans lequel lesdits peptides ont la séquence d'acides aminés:

Arg-Asn-Cys-Lys-Lys-Leu-Gly-Pro-Arg-Glu-Asn-Val-Ala;

et

Pro-Thr-Thr-Ala-Pro-Gln-Thr-Glu-Arg-Met-Met-Arg-Ile-Asn-Trp-Lys-Lys-Trp-Trp-Gln-Val;

respectivement.

14. Vaccin de la revendication 12 ou 13 où le peptide est conjugé à un support macromoléculaire.

15. Vaccin de la revendication 14 dans lequel le support est choisi parmi l'hémocyanine de patelle (KLH), la sérum-albumine de boeuf (SAB), l'ovalbumine (OVA), la bêta-galactosidase, la bêta-pénicillinase, la multipoly-DL-alanyl-poly-L-lysine, la poly-L-lysine, et la protéine VP6 de rotavirus.

16. Peptide selon l'une quelconque des revendications 1-8 ou un vaccin selon l'une quelconque des revendications 9-15 pour utilisation dans le traitement ou la prophylaxie de l'infection à rotavirus chez les mammifères ou les volailles.

**Patentansprüche**

1. Peptid mit einer Aminosäuresequenz entsprechend den Positionen 165-295 des intakten 41 kd neutralisierenden Glykoproteins (VP7) des Rotavirus oder dessen antigenischen Äquivalents, wobei die Peptide ein Molekulargewicht von 14 kd und eine immunologische Aktivität des entsprechenden Antikörpers haben, so daß der intakte Rotavirus neutralisiert wird.

2. Peptid nach Anspruch 1, in welchem die Aminosäuresequenz wie folgt lautet:

**Cys-Asn-Pro-Met-Asp-Ile-Thr-Leu-Tyr-Tyr-Tyr-Gln-Gln**
**-Thr-Asp-Glu-Ala-Asn-Lys-Trp-Ile-Ser-Met-Gly-Ser-Ser-Cys-Thr**
**-Val-Lys-Val-Cys-Pro-Leu-Asn-Thr-Gln-Thr-Leu-Gly-Ile-Gly-Cys**
**-Leu-Ile-Thr-Asn-Pro-Asp-Thr-Phe-Glu-Thr-Val-Ala-Thr-Thr-Glu**
**-Lys-Leu-Val-Ile-Thr-Asp-Val-Val-Asp-Gly-Val-Asn-His-Lys-Leu**
**-Asn-Val-Thr-Thr-Ala-Thr-Cys-Thr-Ile-Arg-Asn-Cys-Lys-Lys-Leu**
**-Gly-Pro-Arg-Glu-Asn-Val-Ala-Ile-Ile-Gln-Val-Gly-Gly-Ala-Asn**
**-Val-Leu-Asp-Ile-Thr-Ala-Asp-Pro-Thr-Thr-Ala-Pro-Gln-Thr-Glu**
**-Arg-Met-Met-Arg-Ile-Asn-Trp-Lys-Lys-Trp-Trp-Gln-Val,**

oder dessen antigenisches Äquivalent.

3. Peptid mit einer Aminosäuresequenz entsprechend den Positionen 40-97 des Nukleokapsidproteins VP6 des Rotavirus oder dessen antigenischen Äquivalents, wobei dieses Peptid ein Molekulargewicht von 6,3 kd und eine immunologische Aktivität des entsprechenden Antikörpers aufweist, so daß der unbeschädigte Rotavirus neutralisiert wird.

4. Peptid nach Anspruch 3 mit folgender Aminosäuresequenz:

**Thr-Met-Asn-Gly-Asn-Phe-Gln-Glu-Thr-Gly-Gly-Ile-**
**Gly-Asn-Leu-Pro-Ile-Arg-Asn-Trp-Asn-Phe-Asn-Phe-Gly-Leu-Leu-**
**Gly-Thr-Thr-Leu-Leu-Asn-Leu-Asp-Ala-Asn-Tyr-Val-Glu-Thr-Ala-**
**Arg-Asn-Thr-Ile-Asp-Tyr-Phe-Val-Asp-Phe-Val-Asp-Asn-Val-Cys-**
**Met;**

oder dessen antigenisches Äquivalent.

5. Peptid mit einer Aminosäuresequenz entsprechend der Positionen 40-60 des Nukleokapsidproteins VP6 des Rotavirus oder dessen antigenischen Äquivalents, wobei das Peptid eine immunologische Aktivität des entsprechenden Antigenes aufweist, die den intakten Rotavirus neutralisiert.

6. Peptid nach Anspruch 5 mit einer Aminosäuresequenz:

**Thr-Met-Asn-Gly-Asn-Glu-Phe-Gln-Thr-Gly-Gly-Ile-Gly-Asn-Leu-Pro-Ile-Arg-Asn-Trp-Asn;**

oder dessen antigenisches Äquivalent.

7. Peptid mit einer Aminosäuresequenz entsprechend den Positionen 232-256 des 84 kd neutralisierenden Glykoproteins (VP3) des Rotavirus oder dessen antigenischen Äquivalents, wobei das Peptid eine immunologische Aktivität des entsprechenden Antikörpers aufweist, so daß der intakte Rotavirus neutralisiert wird.

8. Paptid nach Anspruch 7 mit der Aminosäuresequenz:

**Asn-Ile-Ala-Pro-Ala-Ser-Ile-Val-Ser-Arg-Asn-Ile-Val-Tyr-Thr-Arg-Ala-Gln-Pro-Asn-Gln-Asp-Ile-Ala,**

oder mit der Sequenz:

Asn-Ile-Val-Pro-Val-Ser-Ile-Val-Ser-Arg-Asn-Ile-Val-Tyr-Thr-Arg-Ala-Gln-Pro-Asn-Gln-Asp-Ile-Ala

oder mit dessen antigenen Äquivalent.

9. Impfstoff zur Impfung einer Person zum Schutz gegen eine Rotavirusinfektion mit einem wirksamen Anteil eines Peptids oder einer Mischung von Peptiden gemäß einem der Ansprüche 1-8, wobei der Impfstoff in der Lage ist, Antikörper hervorzurufen, die den intakten Rotorvirus neutralisieren.

10. Impfstoff nach Anspruch 9, in welchem das Peptid an einem Makromolekularträger konjugiert ist.

11. Impfstoff nach Anspruch 10, in welchem der Träger aus folgender Gruppe ausgewählt ist: keyhole limpet hemocyanin (KLH), Rinderserumalbumin (BSA), Ovalbumin (OVA), Beta-glactosidase, Betapenicillinase, Multipoly-DE-alanyl-poly-L-lysin, Poly-L-lysin und VP6 Rotavirusprotein.

12. Impfstoff zur Impfung einer Person zum Schutz gegen die Rotavirusinfektion mit einem wirksamen Anteil eines Peptides oder einer Mischung von Peptiden, wobei der Impfstoff in der Lage ist, Antikörper hervorzurufen, die den intakten Rotavirus neutralisieren, in welchen die Peptide aus einer Gruppe von Peptiden ausgewählt sind, die eine Aminosäuresequenz entsprechend den Positionen 247-259 des intakten VP7 oder dessen antigenischen Äquivalents aufweisen, und der Gruppe der Peptide, die eine Aminosäuresequenz entsprechend den Positionen 275-295 des intakten VP7 oder dessen antigenischen Äquivalents aufweisen.

13. Impfstoff nach Anspruch 13, in welchem die Peptide eine der beiden folgenden Aminosäuresequenzen aufweisen:

**Arg-Asn-Cys-Lys-Lys-Leu-Gly-Pro-Arg-Glu-Asn-Val-Ala**

oder

Pro-Thr-Thr-Ala-Pro-Gln-Thr-Glu-Arg-Met-Met-Arg-Ile-Asn-Trp-Lys-Lys-Trp-Trp-Gln-Val

**14.** Impfstoff nach Anspruch 12 oder 13, in welchem die Peptide mit einem makromolekularen Träger konjungiert sind.

**15.** Impfstoff nach Anspruch 14, in welchem der Träger aus einer Gruppe gewählt ist, die besteht aus keyhole limpet hemocyanin (KLH), Rinderserumslbumin (BSA), Ovalbumin (OVA), Beta-glactosidase, Betapenicillinase, Multipoly-DE-alanyl-poly-L-lysin, Poly-L-lysin und VP6 Rotavirusprotein.

**16.** Peptid nach einem der Ansprüche 1-8 oder Impfstoff entsprechend einem der Ansprüche 9-15 zur Verwendung in der Behandlung oder Prophylaxe der Rotavirusinfektion bei Säugern oder Geflügel.

10 20 30 40 50

HU/5 MET TYR GLY ILE GLU TYR THR THR ILE LEU THR ILE LEU ILE SER ILE ILE LEU LEU ASN TYR ILE LEU LYS THR ILE THR ASN THR MET ASP TYR ILE ILE PHE ARG PHE LEU LEU LEU ILE ALA LEU ILE SER PRO PHE VAL ARG THR
SAII MET TYR GLY ILE GLU TYR THR THR VAL LEU THR PHE LEU ILE SER ILE ILE LEU LEU ASN TYR ILE LEU LYS SER LEU THR ARG ILE MET ASP CYS ILE ILE TYR ARG LEU LEU PHE ILE ILE VAL ILE LEU SER PRO PHE LEU ARG ALA
UK MET TYR GLY ILE GLU TYR THR THR ILE LEU ILE PHE LEU THR SER ILE THR LEU LEU ASN TYR ILE LEU LYS SER ILE THR ARG ILE MET ASP TYR ILE ILE TYR ARG PHE LEU LEU ILE VAL VAL VAL LEU ALA THR MET ILE ARG ALA

60 70 80 90 100

HU/5 GLN ASN TYR GLY MET TYR LEU PRO ILE THR GLY SER LEU ASP ALA VAL TYR THR ASN SER THR SER GLY GLU PRO PHE LEU THR SER THR LEU CYS LEU TYR TYR PRO ALA GLU ALA LYS ASN GLU ILE SER ASP ASP GLU TRP GLU ASN
SAII GLN ASN TYR GLY ILE ASN LEU PRO ILE THR GLY SER MET ASP THR ALA TYR ALA ASN SER THR GLN GLU GLU THR PHE LEU THR SER THR LEU CYS LEU TYR TYR PRO THR GLU ALA ALA THR GLU ILE ASN ASP ASN SER TRP LYS ASP
UK GLN ASN TYR GLY VAL ASN LEU PRO ILE THR GLY SER MET ASP THR ALA TYR ALA ASN SER THR GLN SER GLU PRO PHE LEU THR SER THR LEU CYS LEU TYR TYR PRO VAL GLU ALA SER ASN GLU ILE ALA ASP THR GLU TRP LYS ASP

110 120 130 140 150

HU/5 THR LEU SER GLN LEU PHE LEU THR LYS GLY TRP PRO ILE GLY SER VAL TYR PHE LYS ASP TYR ASN ASP ILE ASN THR PHE SER VAL ASN PRO GLN LEU TYR CYS ASP TYR ASN VAL VAL LEU MET ARG TYR ASP ASN THR SER GLU LEU
SAII THR LEU SER GLN LEU PHE LEU THR LYS GLY TRP PRO THR GLY SER VAL TYR PHE LYS GLU TYR THR ASN ILE ALA SER PHE SER VAL ASP PRO GLN LEU TYR CYS ASP TYR ASN VAL VAL LEU MET LYS TYR ASP ALA THR LEU GLN LEU
UK THR LEU SER GLN LEU PHE LEU THR LYS GLY TRP PRO THR GLY SER VAL TYR PHE LYS GLU TYR THR ASP ILE ALA ALA PHE SER VAL GLU PRO GLN LEU TYR CYS ASP TYR ASN LEU VAL LEU MET LYS TYR ASP SER THR GLN GLU LEU

160 170 180 190 200

HU/5 ASP ALA SER GLU LEU ALA ASP LEU ILE LEU ASN GLU TRP LEU CYS ASN PRO MET ASP ILE SER LEU TYR TYR TYR GLN GLN SER SER GLU SER ASN LYS TRP ILE SER MET GLY THR ASP CYS THR VAL LYS VAL CYS PRO LEU ASN THR
SAII ASP MET SER GLU LEU ALA ASP LEU ILE LEU ASN GLU TRP LEU CYS ASN PRO MET ASP ILE THR LEU TYR TYR TYR GLN GLN THR ASP GLU ALA ASN LYS TRP ILE SER MET GLY SER SER CYS THR ILE LYS VAL CYS PRO LEU ASN THR
UK ASP MET SER GLU LEU ALA ASP LEU ILE LEU ASN GLU TRP LEU CYS ASN PRO MET ASP ILE THR LEU TYR TYR TYR GLN GLN THR ASP GLU ALA ASN LYS TRP ILE SER MET GLY SER SER CYS THR VAL LYS VAL CYS PRO LEU ASN THR
NCDV ASP MET SER GLU LEU ALA ASP LEU ILE LEU ASN GLU TRP LEU CYS ASN PRO MET ASP ILE THR LEU TYR TYR TYR GLN GLN THR ASP GLU ALA ASN LYS TRP ILE SER THR GLY SER SER CYS THR VAL LYS VAL CYS PRO LEU ASN THR

210 220 230 240 250

HU/5 GLN THR LEU GLY ILE GLY CYS LYS THR THR ASP VAL ASN THR PHE GLU ILE VAL ALA SER SER GLU LYS LEU VAL ILE THR ASP VAL VAL ASN GLY VAL ASN HIS ASN ILE ASN ILE SER ILE ASN THR CYS THR ILE ARG ASN CYS ASN
SAII GLN THR LEU GLY ILE GLY CYS LEU THR THR ASP ALA THR THR PHE GLU GLU VAL ALA THR ALA GLU LYS LEU VAL ILE THR ASP VAL VAL ASP GLY VAL ASN HIS LYS LEU ASP VAL THR THR ALA THR CYS THR ILE ARG ASN CYS LYS
UK GLN THR LEU GLY ILE GLY CYS LEU ILE THR ASN PRO ASP THR PHE GLU THR VAL ALA THR THR GLU LYS LEU VAL ILE THR ASP VAL VAL ASP GLY VAL ASN HIS LYS LEU ASN VAL THR THR ALA THR CYS THR ILE ARG ASN CYS LYS
NCDV GLN THR LEU GLY ILE GLY CYS LEU ILE MET ASN PRO ASP THR PHE GLU THR VAL ALA THR THR GLU LYS LEU VAL ILE THR ASP VAL VAL ASP GLY VAL ASN HIS LYS LEU ASN VAL THR THR ALA THR CYS THR ILE ARG ASN CYS LYS

260 270 280 290 300

HU/5 LYS LEU GLY PRO ARG GLU ASN VAL ALA ILE ILE GLN VAL GLY GLY PRO ASN ALA LEU ASP ILE THR ALA ASP PRO THR THR VAL PRO GLN VAL GLN ARG ILE MET ARG ILE ASN TRP LYS LYS TRP TRP GLN VAL PHE TYR THR VAL VAL
SAII LYS LEU GLY PRO ARG GLU ASN VAL ALA VAL ILE GLN VAL GLY GLY SER ASP ILE LEU ASP ILE THR ALA ASP PRO THR THR ALA PRO GLN THR GLU ARG MET MET ARG ILE ASN TRP LYS LYS TRP TRP GLN VAL PHE TYR THR VAL VAL
UK LYS LEU GLY PRO ARG GLU ASN VAL ALA ILE ILE GLN VAL GLY GLY ALA ASN VAL LEU ASP ILE THR ALA ASP PRO THR THR ALA PRO GLN THR GLU ARG MET MET ARG ILE ASN TRP LYS LYS TRP TRP GLN VAL PHE TYR THR VAL VAL
NCDV LYS LEU GLY PRO ARG GLU ASN VAL ALA VAL ILE GLN VAL GLY GLY ALA ASN VAL LEU ASP ILE THR ALA ASP PRO THR THR THR PRO GLN THR GLU ARG MET MET ARG ILE ASN TRP LYS LYS TRP TRP GLN VAL PHE TYR THR VAL VAL

310 320

HU/5 ASP TYR ILE ASN GLN VAL ILE GLN VAL MET SER LYS ARG SER ARG SER LEU ASP ALA ALA ALA PHE TYR TYR ARG ILE
SAII ASP TYR VAL ASP GLN ILE ILE GLN VAL MET SER LYS ARG SER ARG SER LEU ASN SER ALA ALA PHE TYR TYR ARG VAL
UK ASP TYR VAL ASN GLN ILE ILE GLN THR MET SER LYS ARG SER ARG SER LEU ASN SER SER ALA PHE TYR TYR ARG VAL

AMINO ACID SEQUENCE OF ROTAVIRUS UP7 GYLCOPROTEIN (The 14K polypeptide is shown in the box)

FIG. 1

MET ASP VAL LEU TYR SER LEU SER LYS THR LEU LYS ASP ALA 14
5'- GGCTTTTAAACGAAGTCTTCAAC ATG GAT GTC CTA TAC TCT TTG TCA AAG ACT CTT AAA GAC GCT 65

ARG ASP LYS ILE VAL GLU GLY THR LEU TYR SER ASN VAL SER ASP LEU ILE GLN GLN PHE 34
AGA GAC AAA ATT GTC GAA GGC ACA TTG TAT TCT AAC GTG AGT GAT CTA ATT CAA CAA TTT 125

ASN GLN MET ILE ILE THR MET ASN GLY ASN GLU PHE GLN THR GLY GLY ILE GLY ASN LEU 54
AAT CAA ATG ATA ATT ACT ATG AAT GGA AAT GAA TTT CAA ACT GGA GGA ATC GCT AAT TTG 185

PRO ILE ARG ASN TRP ASN PHE ASN PHE GLY LEU LEU GLY THR THR LEU LEU ASN LEU ASP 74
CCA ATT AGA AAC TGG AAT TTT AAT TTC GGG TTA CTT GGA ACA ACT TTG CTG AAC TTA GAC 245

ALA ASN TYR VAL GLU THR ALA ARG ASN THR ILE ASP TYR PHE VAL ASP PHE VAL ASP ASN 94
GCT AAT TAT GTT GAA ACG GCA AGA AAT ACA ATT GAT TAT TTC GTG GAT TTT GTA GAC AAT 305

VAL CYS MET ASP GLU MET VAL ARG GLU SER GLN ARG ASN GLY ILE ALA PRO GLN SER ASP 114
GTA TGC ATG GAT GAG ATG GTT AGA GAA TCA CAA AGG AAC GGA ATT GCA CCT CAA TCA GAC 365

SER LEU ARG LYS LEU SER ALA ILE LYS PHE LYS ARG ILE ASN PHE ASP ASN SER SER GLU 134
TCC CTA AGA AAG CTG TCA GCC ATT AAA TTC AAA AGA ATA AAT TTT GAT AAT TCG TCG GAA 425

TYR ILE GLU ASN TRP ASN LEU GLN ASN ARG ARG GLN ARG THR GLY PHE THR PHE HIS LYS 154
TAC ATA GAA AAC TGG AAT TTG CAA AAT AGA AGA CAG AGG ACA GGT TTC ACT TTT CAT AAA 485

PRO ASN ILE PHE PRO TYR SER ALA SER PHE THR LEU ASN ARG SER GLN PRO ALA HIS ASP 174
CCA AAC ATT TTT CCT TAT TCA GCA TCA TTT ACA CTA AAT AGA TCA CAA CCC GCT CAT GAT 545

ASN LEU MET GLY THR MET TRP LEU ASN ALA GLY SER GLU ILE GLN VAL ALA GLY PHE ASP 194
AAT TTG ATG GGC ACA ATG TGG TTA AAC GCA GGA TCG GAA ATT CAA GTC GCT GGA TTT GAC 605

TYR SER CYS ALA ILE ASN ALA PRO ALA ASN ILE GLN GLN PHE GLU HIS ILE VAL PRO LEU 214
TAC TCA TGT GCT ATT AAC GCA CCA GCC AAT ATA CAA CAA TTT GAG CAT ATT GTG CCA CTC 665

ARG ARG VAL LEU THR THR ALA THR ILE THR LEU LEU PRO ASP ALA GLU ARG PHE SER PHE 234
CGA AGA GTG TTA ACT ACA GCT ACG ATA ACT CTT CTA CCA GAC GCG GAA AGG TTT AGT TTT 725

PRO ARG VAL ILE ASN SER ALA ASP GLY ALA THR THR TRP PHE PHE ASN PRO VAL ILE LEU 254
CCA AGA GTG ATC AAT TCA GCT GAC GGC GCA ACT ACA TGG TTT TTC AAC CCA GTG ATT CTC 785

ARG PRO ASN ASN VAL GLU VAL GLU PHE LEU LEU ASN GLY GLN ILE ILE ASN THR TYR GLN 274
AGG CCG AAT AAC GTT GAA GTG GAG TTT CTA TTG AAT GGA CAG ATA ATA AAC ACT TAT CAA 845

ALA ARG PHE GLY THR ILE VAL ALA ARG ASN PHE ASP THR ILE ARG LEU SER PHE GLN LEU 294
GCA AGA TTT GGA ACT ATC GTA GCT AGA AAT TTT GAT ACT ATT AGA CTA TCA TTC CAG TTA 905

MET ARG PRO PRO ASN MET THR PRO ALA VAL ALA VAL LEU PHE PRO ASN ALA GLN PRO PHE 314
ATG AGA CCA CCA AAC ATG ACA CCA GCA GTA GCA GTA CTA TTC CCG AAT GCA CAG CCA TTC 965

GLU HIS HIS ALA THR VAL GLY LEU THR LEU ARG ILE GLU SER ALA VAL CYS GLU SER VAL 334
GAA CAT CAT GCA ACA GTG GGA TTG ACA CTT AGA ATT GAG TCT GCA GTT TGT GAG TCT GTA 1025

LEU ALA ASP ALA SER GLU THR LEU LEU ALA ASN VAL THR SER VAL ARG GLN GLU TYR ALA 354
CTC GCC GAT GCA AGT GAA ACT CTA TTA GCA AAT GTA ACA TCC GTT AGG CAA GAG TAC GCA 1085

ILE PRO VAL GLY PRO VAL PHE PRO PRO GLY MET ASN TRP THR ASP LEU ILE THR ASN TYR 374
ATA CCA GTT GGA CCA GTC TTT CCA CCA GGT ATG AAC TGG ACT GAT TTA ATC ACC AAT TAT 1145

SER PRO SER ARG GLU ASP ASN LEU GLN ARG VAL PHE THR VAL ALA SER ILE ARG SER MET 394
TCA CCG TCT AGG GAG GAC AAT TTG CAA CGC GTA TTT ACA GTG GCT TCC ATT AGA AGC ATG 1205

LEU ILE LYS ººº 397
CTC ATT AAA TGA GGACCAAGCTAACAACTTGGTATCCAACTTTGGTGAGTATGTAGCTATATCAAGCTGTTTGAA 1280

CTCTGTAAGTAAGGATGCGTATACGCATTCGCTACACTGAGTTAATCACTCTGATGGTATAGTGAGAGGATGTGACC-3' 1357

FIG. 2

| | | | | | | |
|---|---|---|---|---|---|---|
| C486 (bovine) 1 | MASLIYRQLL | TNSYTVELSD | EIQEIGSTKT | QNVTVNPGPF | AQTNYASVNW | GPGETNDSTT |
| SA-11 (simian) 1 | LIYRQLL | TNSYTVELSD | EIQEIGSTKT | QNVTVNPGPF | AQTNYAPVNW | GPGETNDSTT |
| 61 | VEPVLDGPYQ | PTTFNPPVSY | WMLLAPTNAG | VVDQGTNNTN | RWLATILIKP | NVQQVERTYT |
| 61 | VEPVLDGPYQ | PTTFNPPVSY | WMLLAPTNAG | VVVEGTNNTN | RWLATILIEP | NVQQVERTYT |
| 121 | LFGQQVQVTV | SNDSQTKWKF | VDLSKQTQDG | NYSQHGPLLS | TPKLYGVMKH | GGKIYTYNGE |
| 121 | LFGQQVQVTV | SNDSQTKWKF | VDLSKQTQDG | NYSQHGSLLS | TPKLYGVMKH | GGKIYTYNGE |
| 181 | TPNATTGYYS | TTNFDTVNMT | AYCDFYIIPL | AQEAKCTEYI | NNGLPPIQNT | RNIVPVSIVS |
| 181 | TPNANTGYYS | TTNFDTVNMT | AYCDFYIIPL | AQEAKCTEYI | NNGLPPIQNT | RNIVPVSIVS |
| 241 | RNIVYTRAQP | NQDIVVSKTS | LWKEMQYNRD | IVIRFKFANS | IIKSGGLGYK | WSEVSFKPAN |
| 241 | RNIVYTRAQP | NQDIVVSKTS | LWKEMQYNRD | IVIRFKFANS | IIKSGGLGYK | WSEVSFKPAF |
| 301 | YQYTYTRDGE | EVTAHTTCSV | NGINDFNYNG | GSLPTDFVIS | KYEVIKENSF | VYIDYWDDSQ |
| 301 | YQYTYTRDGE | EVTAHTTCSV | NGVNDFNYNG | GSLPTDFVIS | KYEVIKENSF | VYIDYWDDSQ |
| 361 | AFRNMVYVRS | LAADLNSVMC | TGGDYSFAIP | VGNYPVMTGG | AVSLHSAGVT | LSTQFTDFVS |
| 361 | AFRNMVYVRS | LAADLNSVMC | TGGDYSFALP | VGNYPVMTGG | AVSLHSAGVT | LSTQFTDFVS |
| 421 | LNSLRFRFRL | SVEEPPFSIL | RTRVSGLYGL | PAAKPNNSQE | YYEIAGRFSL | ISLVPSNDDY |
| 421 | LNSLRFRFRL | SVEEPPFSIL | RTRVSGLYGL | PAAKPNNSQE | YYEIAGRFSL | ISLVPLNDDY |
| 481 | QTPIINSVTV | RQDLERQLGE | LRDEFNNLSQ | QIAMSQLIDL | ALLPLDMFSM | FSGIKSTIDA |
| 481 | QTPIMNSVTV | RQDLERQLGE | LRDEFNNLSQ | QIAMSQLIDL | ALLPLDMFSM | FSGIKSTIDA |
| 541 | AKSMATNVMK | RFKKSSLANS | VSTLTDSLSD | AASSISRSAS | VRSVSSTASA | WTEVSNITSD |
| 541 | AKSMATNVMK | RFKKSSLANS | VSTLTDSLSD | AASSISRSAS | VRSVSSTASA | WTEVSNIASD |
| 601 | INVTTSSIST | QTSTISRRLR | LKEMATQTDG | MNFDDISAAV | LKTKIDKSTQ | LNTNTLPEIV |
| 601 | INVTTSSIST | QTSTISRRLR | LKEMATQTDG | MNFDDISAAV | LKTKIDKSTQ | LNTNTLPEIV |
| 661 | TEASEKFIPN | RAYRVIKDDE | VLEASTDGKY | FAYKVETILK | RFHSMYKFAD | LVTDSPVISA |
| 661 | TEASEKFIPN | RAYRVIKDDE | VLEASIDGKY | FAYKVETFEE | IPFDVQKFAD | LVTDSPVISA |
| 721 | IIDFKTLKNL | NDNYGISRQQ | ALNLLRSDPR | VLREFINQDN | PIIRNRIESL | IMQCRL |
| 721 | IIDFKTLKNL | NDNYGISRQQ | ALNLLRSDPR | | | |

AMINO ACID SEQUENCE OF ROTAVIRUS VP3 PROTEIN

| ALMINO ACID | THREE LETTER SYMBOL | ONE LETTER SYMBOL |
|---|---|---|
| ALANINE | Ala | A |
| ARGININE | Arg | R |
| ASPARAGINE | Asn | N |
| ASPARTIC ACID | Asp | D |
| ASN AND/OR ASP | Asx | B |
| CYSTEINE | Cys | C |
| GLUTAMINE | Gln | Q |
| GLUTAMIC ACID | Glu | E |
| GLN AND/OR GLU | Glx | Z |
| GLYCINE | Gly | G |
| HISTIDINE | His | H |
| ISOLEUCINE | Lie | I |
| LEUCINE | Leu | L |
| LYSINE | Lys | K |
| METHIONINE | Met | M |
| PHENYLALANINE | Phe | F |
| PROLINE | Pro | P |
| SERINE | Ser | S |
| THREONINE | Thr | T |
| TRYPTOPHAN | Trp | W |
| TYROSINE | Tyr | Y |
| VALINE | Val | V |

KEY

FIG. 3

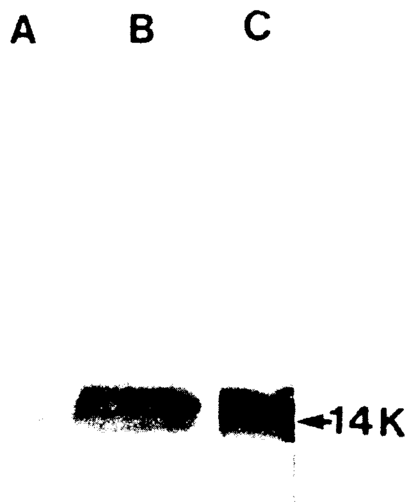


FIG.4

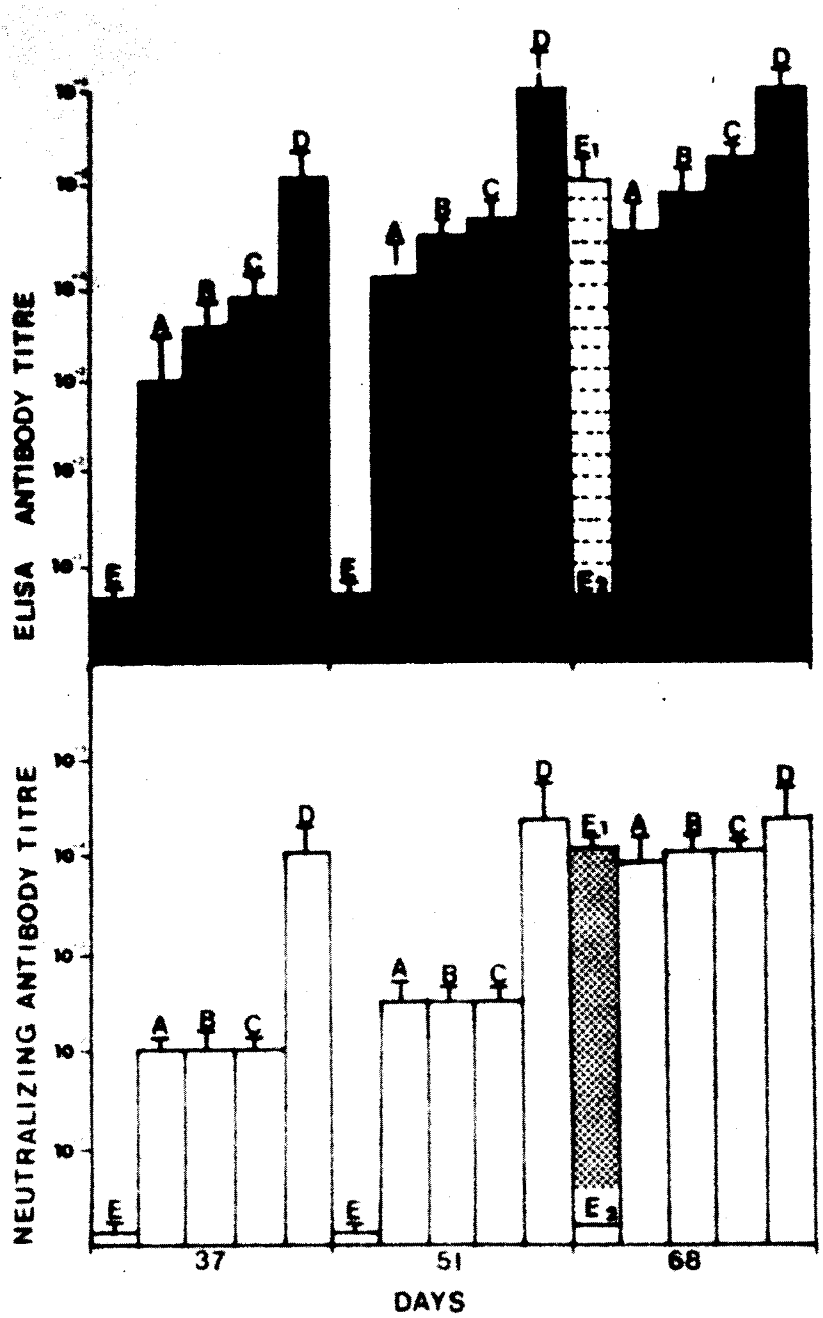
ELISA ANTIBODY TITRE
NEUTRALIZING ANTIBODY TITRE
A
B
C
D
E
E1
E2
37
51
68
DAYS

FIG. 5

PROTEIN PROFILE

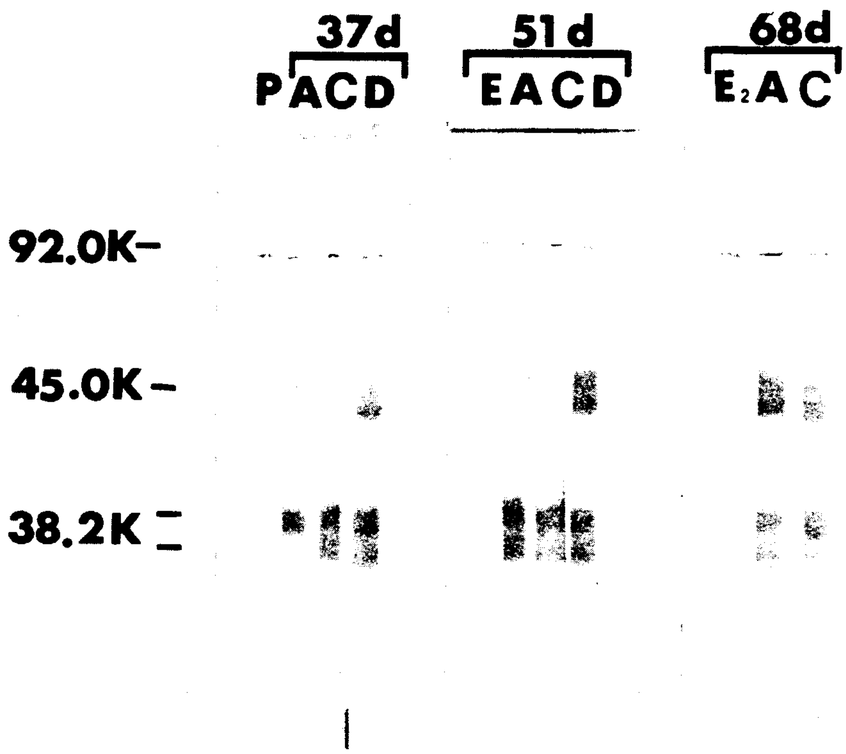


FIG.6

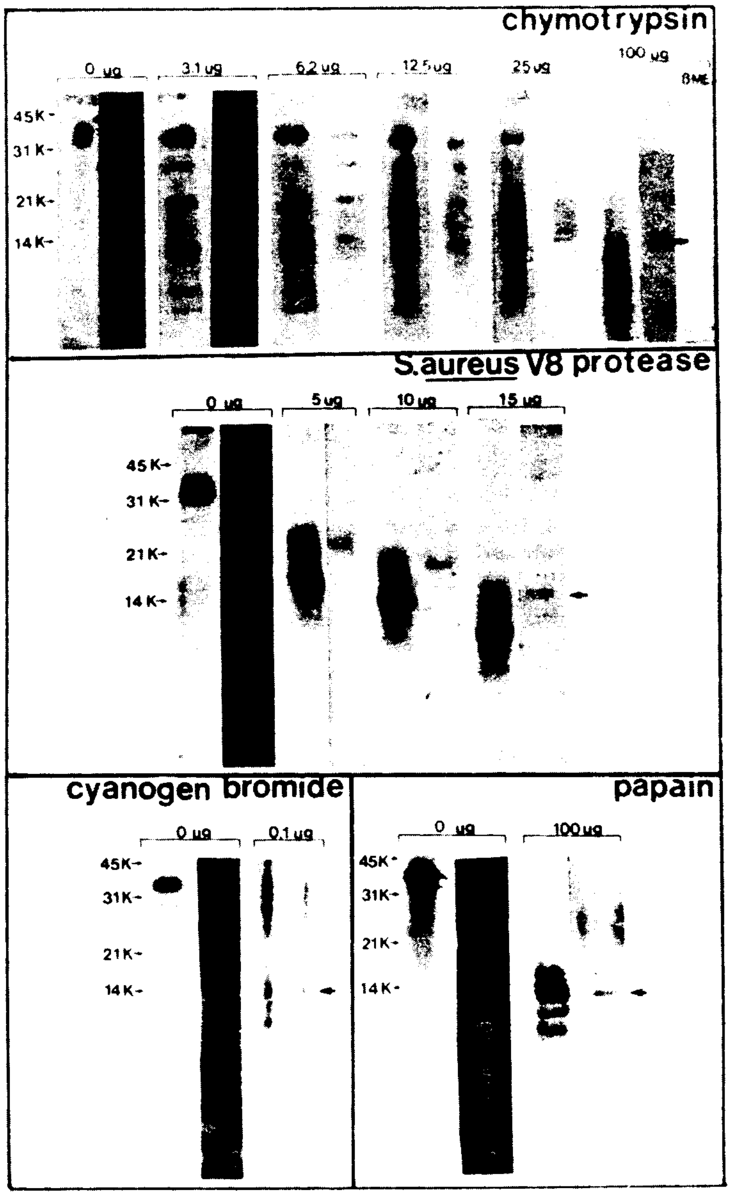
chymotrypsin
0 ug
3.1 ug
6.2 ug
12.5 ug
25 ug
100 ug
BME
45K
31K
21K
14K
S.aureus V8 protease
0 ug
5 ug
10 ug
15 ug
45K
31K
21K
14K
cyanogen bromide
0 ug
0.1 ug
45K
31K
21K
14K
papain
0 ug
100 ug
45K
31K
21K
14K

FIG. 7

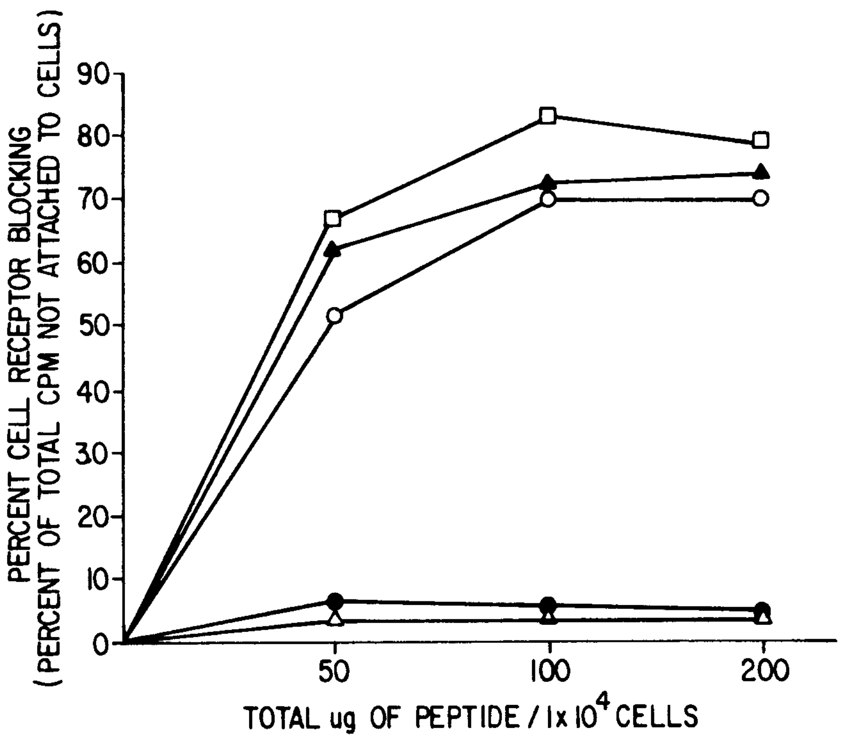
PERCENT CELL RECEPTOR BLOCKING
(PERCENT OF TOTAL CPM NOT ATTACHED TO CELLS)
90
80
70
60
50
40
30
20
10
0
50
100
200
TOTAL ug OF PEPTIDE / 1x10⁴ CELLS

FIG. 8

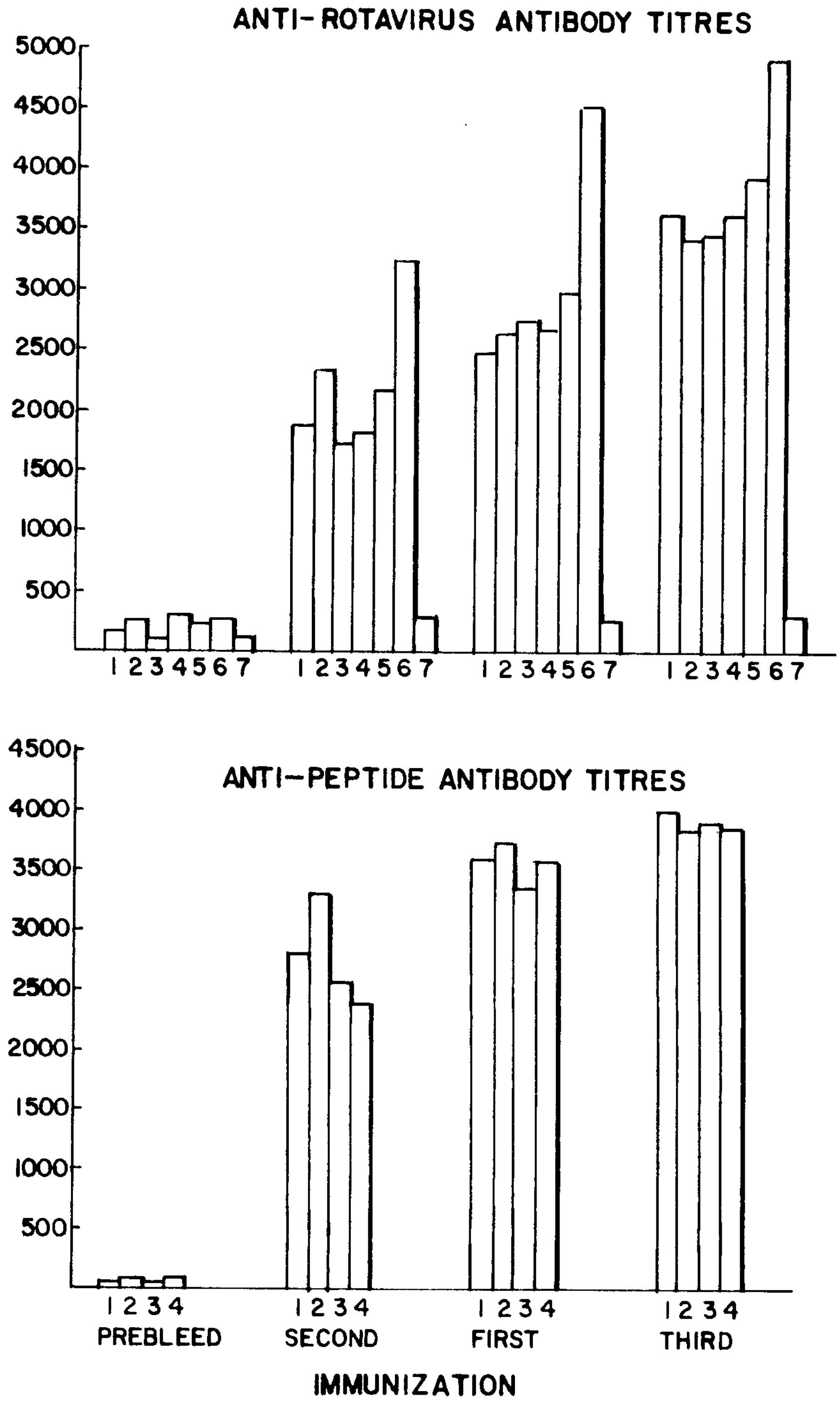


ELISA ANTIBODY TITERS TO SYNTHETIC PEPTIDES

FIG. 9

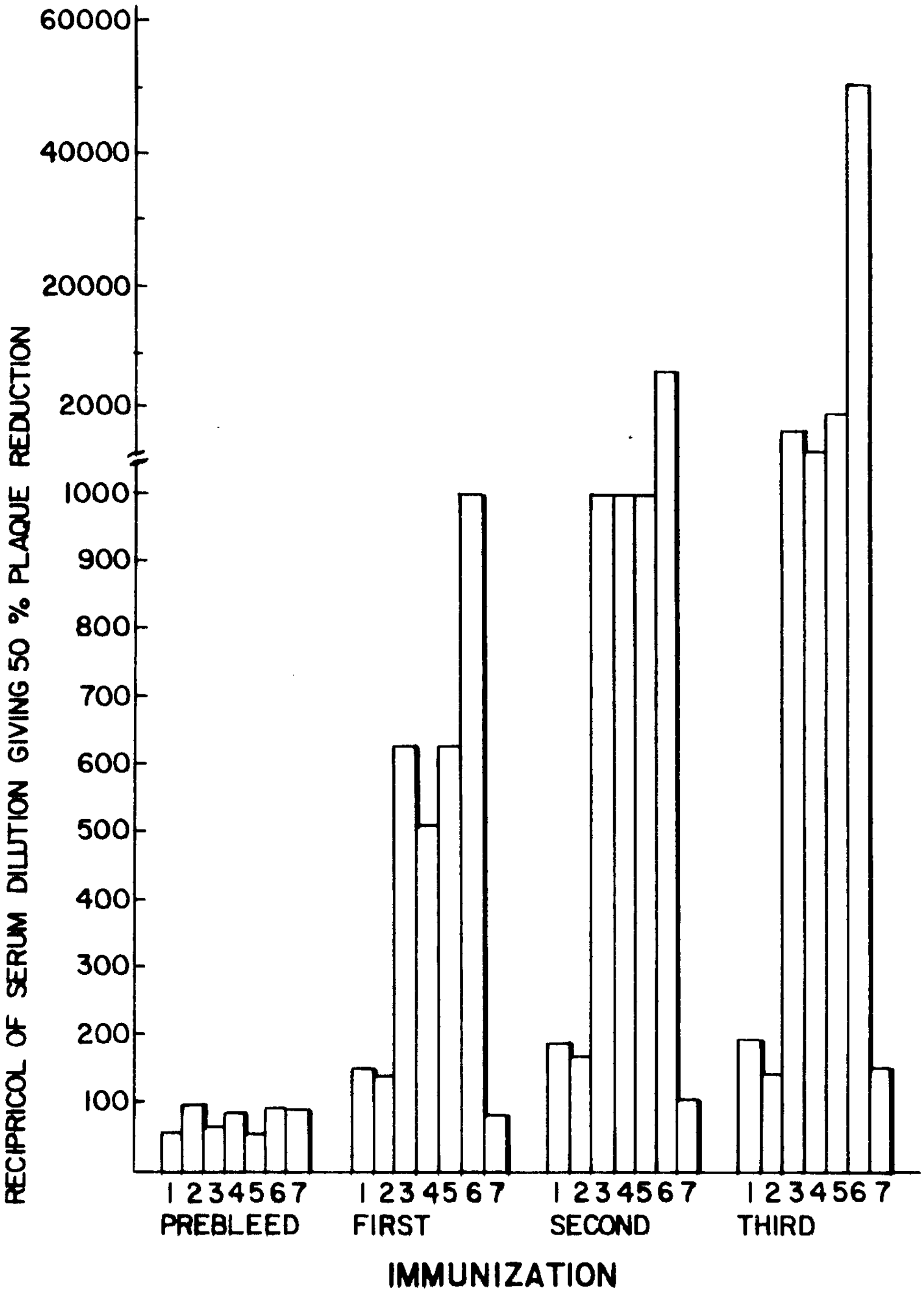


VIRUS NEUTRALIZING ANTIBODY TITRES INDUCED IN MICE BY SYNTHETIC PEPTIDES.

FIG. 10

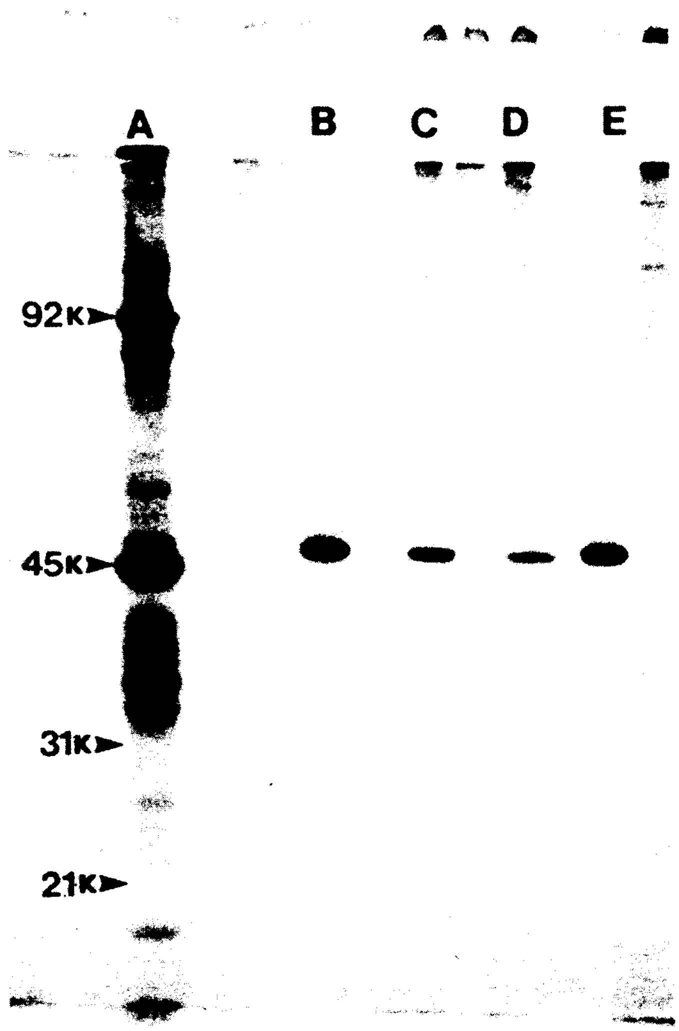


FIG. 11

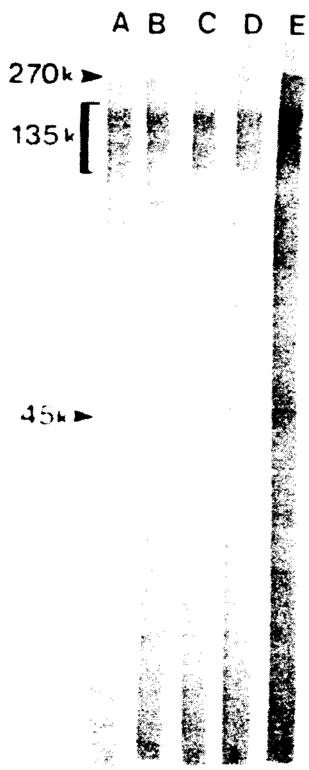


FIG. 12

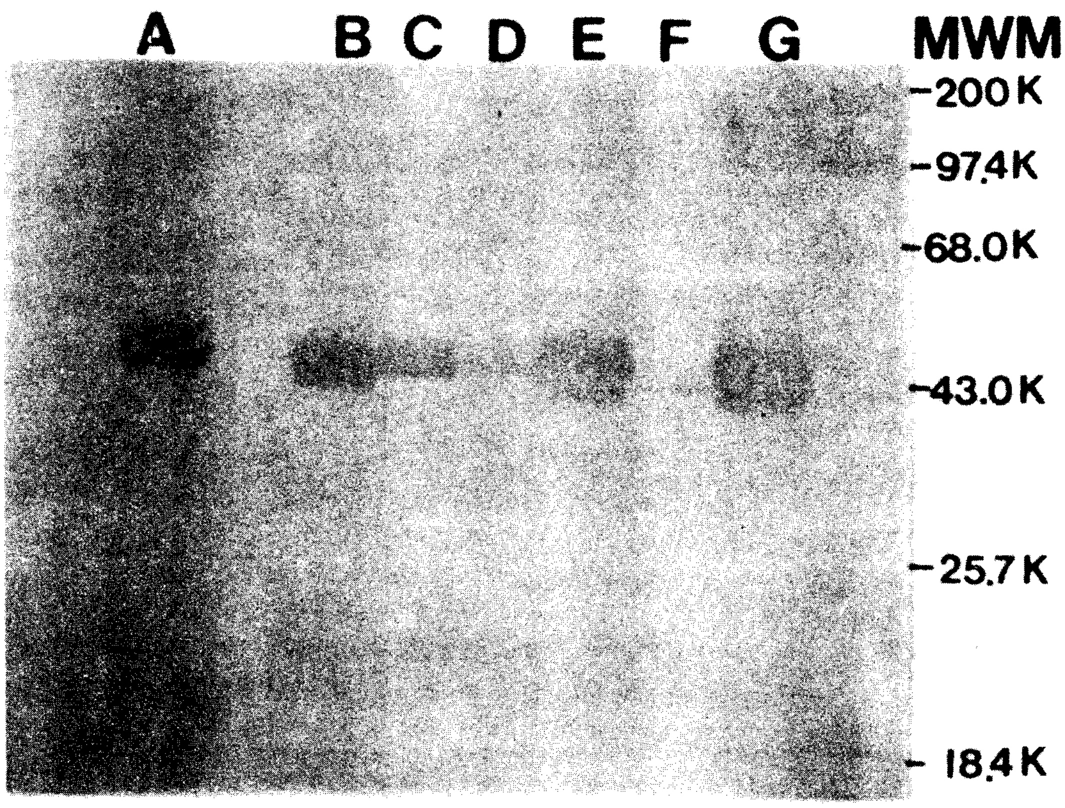


FIG. 13

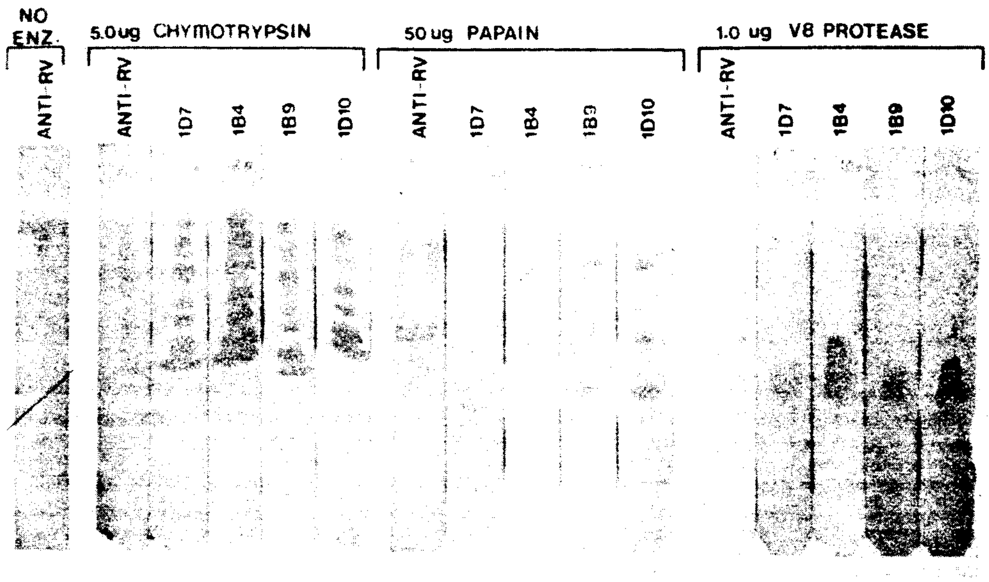
NO ENZ.
5.0ug CHYMOTRYPSIN
50ug PAPAIN
1.0 ug V8 PROTEASE
ANTI-RV
ANTI-RV
1D7
1B4
1B9
1D10
ANTI-RV
1D7
1B4
1B9
1D10
ANTI-RV
1D7
1B4
1B9
1D10

FIG.14

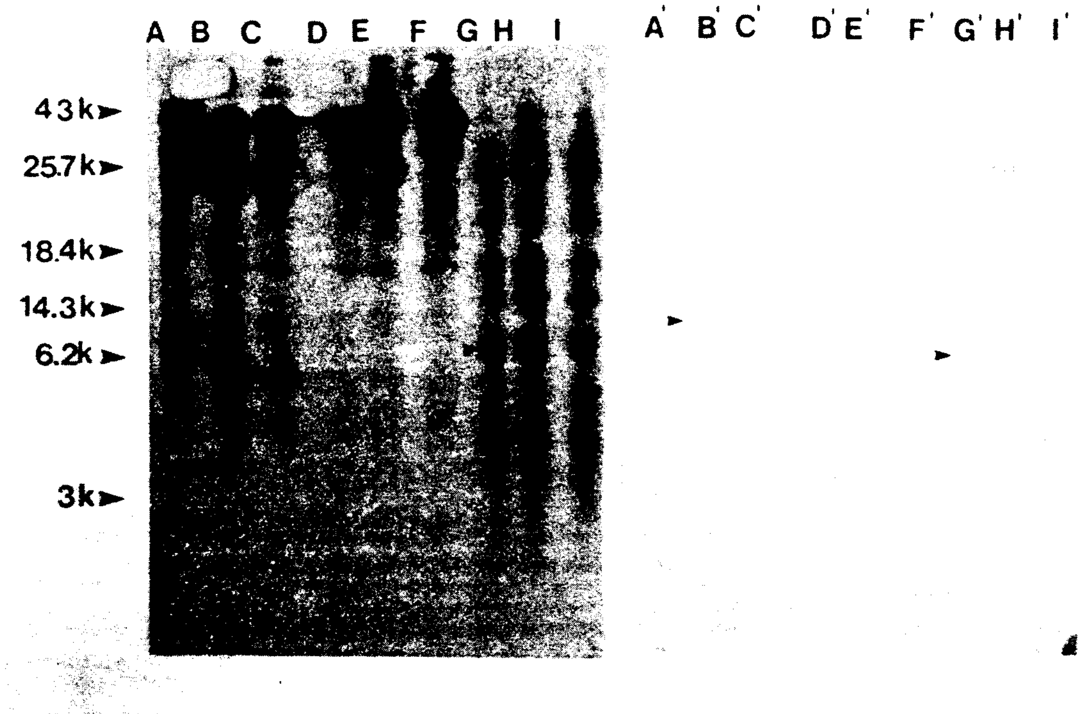


FIG. 15

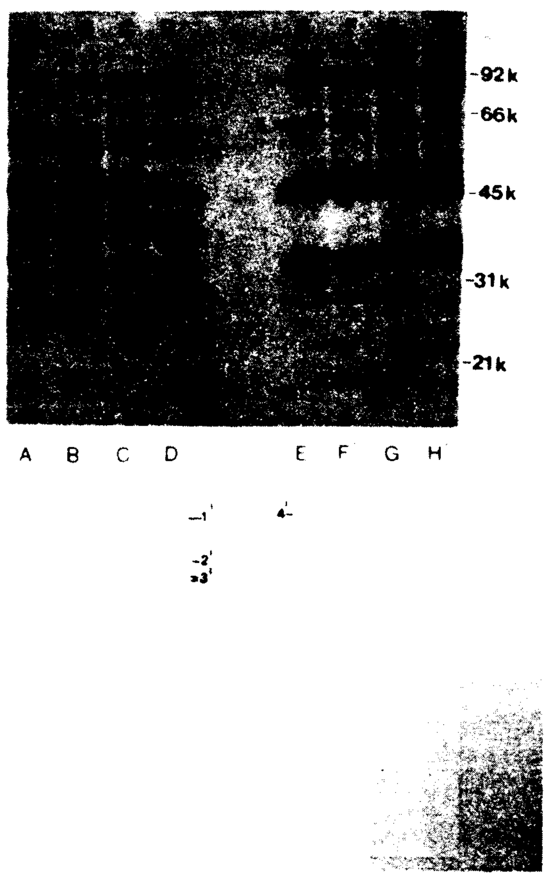


FIG.16

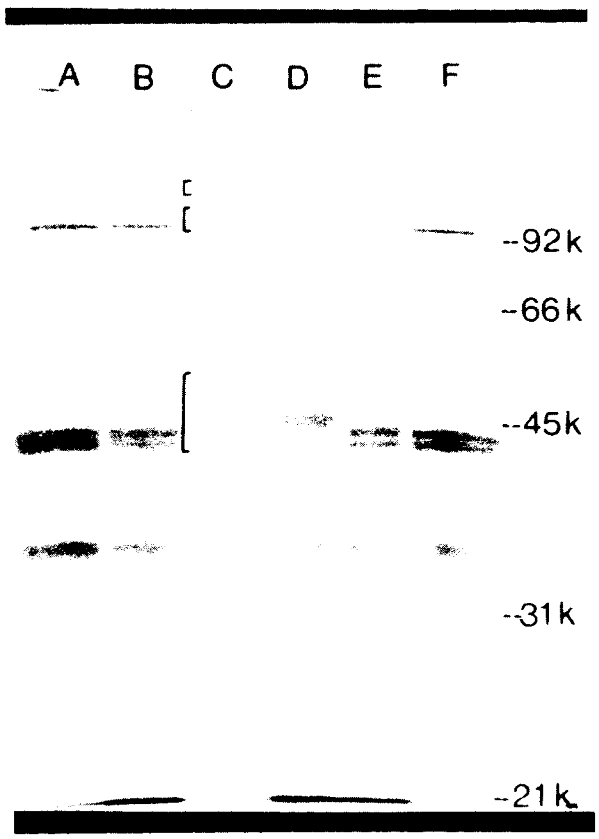


FIG.17

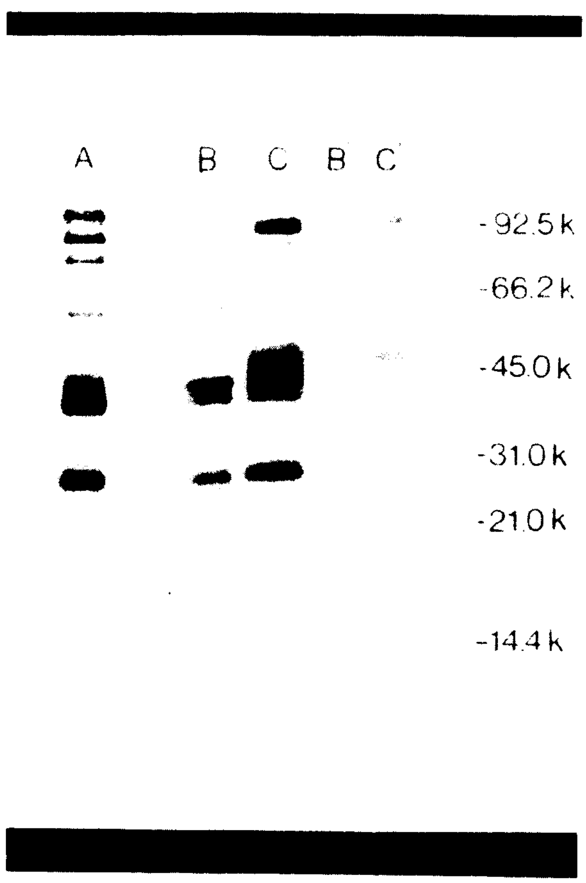


FIG.18

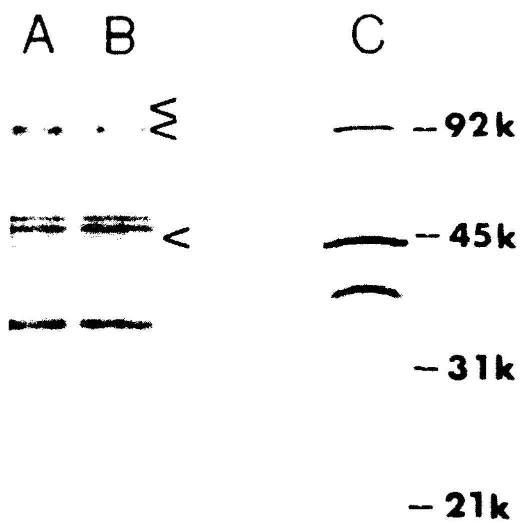


FIG.19

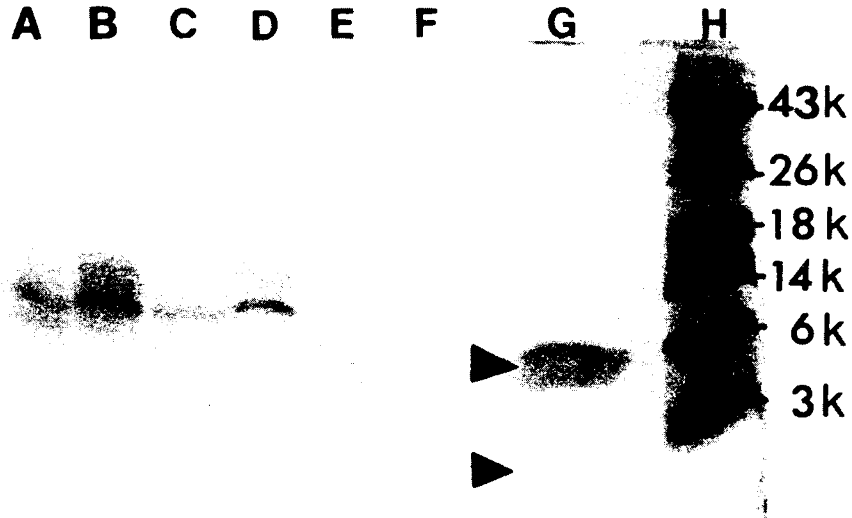


FIG.20

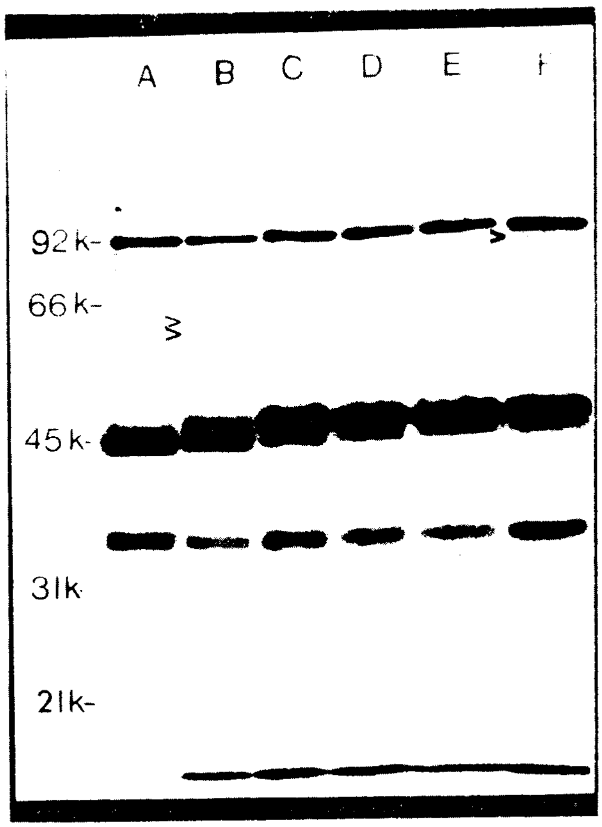


FIG. 21

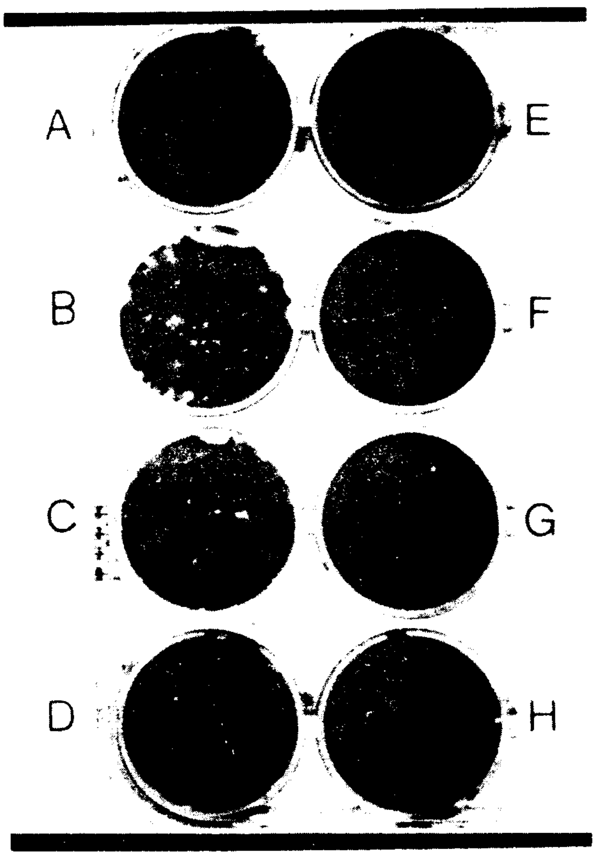


FIG.22